# EUROPEAN PATENT APPLICATION

(11) **EP 3 998 284 A1**
(43) Date of publication of application: **18.05.2022**
(21) Application number: 20837734.1
(22) Date of filing: 13.07.2020
(51) Int. Cl.: C07K 16/28, A61K 47/68, A61P 35/00, A61K 39/00

(54) **COMPOSITION FOR PREVENTING, AMELIORATING OR TREATING IMMUNE CHECKPOINT INHIBITOR-RESISTANT CANCER**

(30) Priority: 11.07.2019 KR 20190084098
(71) Applicant: Good T Cells, Inc., Seoul 03722 (KR)
(72) Inventor: KIM, Beom Seok, Seoul 04029 (KR); KIM, Jung Ho, Seoul 04136 (KR)
(74) Representative: Schnappauf, Georg
(86) International application number: PCT/KR2020/009181
(87) International publication number: WO 2021/006714

(57) **Abstract**

The present invention relates to a composition for preventing, ameliorating or treating immune checkpoint inhibitor-resistant cancer, comprising, as an active ingredient, a binding molecule capable of specifically binding to leucine-rich and immunoglobulin-like domains 1 (Lrig-1) protein, which is a protein present on the surface of regulatory T cells (Treg). The binding molecule specific to the Lrig-1 protein according to the present invention suppresses the function of regulatory T cells in which the Lrig-1 protein is expressed at high levels, such as regulatory T cells in tumor infiltrating lymphocytes (TIL) or activated regulatory T cells, thereby very effectively preventing a phenomenon in which the function of effective T cells is suppresses by the regulatory T cells. Accordingly, the binding molecule specific to the Lrig-1 protein can effectively prevent, ameliorate or treat immune-evasive cancer, that is, cancer with resistance to immune checkpoint inhibitors.

<110>    Good T Cells, Inc.

<120>    A composition for preventing, alleviating or treating immune
         checkpoint inhibitor resistant cancer

<130>    POPB204125PCT

<150>    KR 10-2019-0084098
<151>    2019-07-11

<160>    91

<170>    KoPatentIn 3.0

<210>    1
<211>    759
<212>    PRT
<213>    Homo sapiens

<400>    1
Gly Pro Arg Ala Pro Cys Ala Ala Ala Cys Thr Cys Ala Gly Asp Ser
  1               5                  10                  15

Leu Asp Cys Gly Gly Arg Gly Leu Ala Ala Leu Pro Gly Asp Leu Pro
             20                  25                  30

Ser Trp Thr Arg Ser Leu Asn Leu Ser Tyr Asn Lys Leu Ser Glu Ile
         35                  40                  45

Asp Pro Ala Gly Phe Glu Asp Leu Pro Asn Leu Gln Glu Val Tyr Leu
     50                  55                  60

Asn Asn Asn Glu Leu Thr Ala Val Pro Ser Leu Gly Ala Ala Ser Ser
 65                  70                  75                  80

His Val Val Ser Leu Phe Leu Gln His Asn Lys Ile Arg Ser Val Glu
                 85                  90                  95

Gly Ser Gln Leu Lys Ala Tyr Leu Ser Leu Glu Val Leu Asp Leu Ser
            100                 105                 110

Leu Asn Asn Ile Thr Glu Val Arg Asn Thr Cys Phe Pro His Gly Pro
        115                 120                 125

Pro Ile Lys Glu Leu Asn Leu Ala Gly Asn Arg Ile Gly Thr Leu Glu
    130                 135                 140

Leu Gly Ala Phe Asp Gly Leu Ser Arg Ser Leu Leu Thr Leu Arg Leu
145                 150                 155                 160

Ser Lys Asn Arg Ile Thr Gln Leu Pro Val Arg Ala Phe Lys Leu Pro
                165                 170                 175

Arg Leu Thr Gln Leu Asp Leu Asn Arg Asn Arg Ile Arg Leu Ile Glu
            180                 185                 190

Gly Leu Thr Phe Gln Gly Leu Asn Ser Leu Glu Val Leu Lys Leu Gln
        195                 200                 205

Arg Asn Asn Ile Ser Lys Leu Thr Asp Gly Ala Phe Trp Gly Leu Ser
    210                 215                 220

Lys Met His Val Leu His Leu Glu Tyr Asn Ser Leu Val Glu Val Asn

225                 230                 235                 240

Ser Gly Ser Leu Tyr Gly Leu Thr Ala Leu His Gln Leu His Leu Ser
                245                 250                 255

Asn Asn Ser Ile Ala Arg Ile His Arg Lys Gly Trp Ser Phe Cys Gln
            260                 265                 270

Lys Leu His Glu Leu Val Leu Ser Phe Asn Asn Leu Thr Arg Leu Asp
        275                 280                 285

Glu Glu Ser Leu Ala Glu Leu Ser Ser Leu Ser Val Leu Arg Leu Ser
    290                 295                 300

His Asn Ser Ile Ser His Ile Ala Glu Gly Ala Phe Lys Gly Leu Arg
305                 310                 315                 320

Ser Leu Arg Val Leu Asp Leu Asp His Asn Glu Ile Ser Gly Thr Ile
                325                 330                 335

Glu Asp Thr Ser Gly Ala Phe Ser Gly Leu Asp Ser Leu Ser Lys Leu
            340                 345                 350

Thr Leu Phe Gly Asn Lys Ile Lys Ser Val Ala Lys Arg Ala Phe Ser
        355                 360                 365

Gly Leu Glu Gly Leu Glu His Leu Asn Leu Gly Gly Asn Ala Ile Arg
    370                 375                 380

Ser Val Gln Phe Asp Ala Phe Val Lys Met Lys Asn Leu Lys Glu Leu
385                 390                 395                 400

His Ile Ser Ser Asp Ser Phe Leu Cys Asp Cys Gln Leu Lys Trp Leu
                405                 410                 415

Pro Pro Trp Leu Ile Gly Arg Met Leu Gln Ala Phe Val Thr Ala Thr
            420                 425                 430

Cys Ala His Pro Glu Ser Leu Lys Gly Gln Ser Ile Phe Ser Val Pro
        435                 440                 445

Pro Glu Ser Phe Val Cys Asp Asp Phe Leu Lys Pro Gln Ile Ile Thr
    450                 455                 460

Gln Pro Glu Thr Thr Met Ala Met Val Gly Lys Asp Ile Arg Phe Thr
465                 470                 475                 480

Cys Ser Ala Ala Ser Ser Ser Ser Ser Pro Met Thr Phe Ala Trp Lys
                485                 490                 495

Lys Asp Asn Glu Val Leu Thr Asn Ala Asp Met Glu Asn Phe Val His
            500                 505                 510

Val His Ala Gln Asp Gly Glu Val Met Glu Tyr Thr Thr Ile Leu His
        515                 520                 525

Leu Arg Gln Val Thr Phe Gly His Glu Gly Arg Tyr Gln Cys Val Ile
    530                 535                 540

Thr Asn His Phe Gly Ser Thr Tyr Ser His Lys Ala Arg Leu Thr Val
545                 550                 555                 560

Asn Val Leu Pro Ser Phe Thr Lys Thr Pro His Asp Ile Thr Ile Arg

                565                 570                 575

Thr Thr Thr Val Ala Arg Leu Glu Cys Ala Ala Thr Gly His Pro Asn
            580                 585                 590

Pro Gln Ile Ala Trp Gln Lys Asp Gly Gly Thr Asp Phe Pro Ala Ala
        595                 600                 605

Arg Glu Arg Arg Met His Val Met Pro Asp Asp Asp Val Phe Phe Ile
    610                 615                 620

Thr Asp Val Lys Ile Asp Asp Ala Gly Val Tyr Ser Cys Thr Ala Gln
625                 630                 635                 640

Asn Ser Ala Gly Ser Ile Ser Ala Asn Ala Thr Leu Thr Val Leu Glu
                645                 650                 655

Thr Pro Ser Leu Val Val Pro Leu Glu Asp Arg Val Val Ser Val Gly
            660                 665                 670

Glu Thr Val Ala Leu Gln Cys Lys Ala Thr Gly Asn Pro Pro Pro Arg
        675                 680                 685

Ile Thr Trp Phe Lys Gly Asp Arg Pro Leu Ser Leu Thr Glu Arg His
    690                 695                 700

His Leu Thr Pro Asp Asn Gln Leu Leu Val Val Gln Asn Val Val Ala
705                 710                 715                 720

Glu Asp Ala Gly Arg Tyr Thr Cys Glu Met Ser Asn Thr Leu Gly Thr
                725                 730                 735

Glu Arg Ala His Ser Gln Leu Ser Val Leu Pro Ala Ala Gly Cys Arg
            740                 745                 750

Lys Asp Gly Thr Thr Val Gly
        755

<210> 2
<211> 2397
<212> DNA
<213> Homo sapiens

<400> 2
ggcccgcggg cgccctgcgc ggccgcctgc acttgcgctg gggactcgct ggactgcggt 60

gggcgcgggc tggctgcgtt gcccggggac ctgccctcct ggacgcggag cctaaacctg 120

agttacaaca aactctctga gattgaccct gctggttttg aggacttgcc gaacctacag 180

gaagtgtacc tcaataataa tgagttgaca gcggtaccat ccctgggcgc tgcttcatca 240

catgtcgtct ctctctttct gcagcacaac aagattcgca gcgtggaggg gagccagctg 300

aaggcctacc tttccttaga agtgttagat ctgagtttga acaacatcac ggaagtgcgg 360

aacacctgct ttccacacgg accgcctata aaggagctca acctggcagg caatcggatt 420

ggcaccctgg agttgggagc atttgatggt ctgtcacggt cgctgctaac tcttcgcctg 480

agcaaaaaca ggatcaccca gcttcctgta agagcattca agctacccag gctgacacaa 540

ctggacctca atcggaacag gattcggctg atagagggcc tcaccttcca ggggctcaac 600

agcttggagg tgctgaagct tcagcgaaac aacatcagca aactgacaga tggggccttc 660

tggggactgt ccaagatgca tgtgctgcac ctggagtaca acagcctggt agaagtgaac 720

agcggctcgc tctacggcct cacggccctg catcagctcc acctcagcaa caattccatc 780

gctcgcattc accgcaaggg ctggagcttc tgccagaagc tgcatgagtt ggtcctgtcc 840

ttcaacaacc tgacacggct ggacgaggag agcctggccg agctgagcag cctgagtgtc 900

ctgcgtctca gccacaattc catcagccac attgcggagg gtgccttcaa gggactcagg 960

agcctgcgag tcttggatct ggaccataac gagatttcgg gcacaataga ggacacgagc 1020

ggcgccttct cagggctcga cagcctcagc aagctgactc tgtttggaaa caagatcaag 1080

tctgtggcta agagagcatt ctcggggctg gaaggcctgg agcacctgaa ccttggaggg 1140

aatgcgatca gatctgtcca gtttgatgcc tttgtgaaga tgaagaatct taaagagctc 1200

catatcagca gcgacagctt cctgtgtgac tgccagctga agtggctgcc cccgtggcta 1260

attggcagga tgctgcaggc ctttgtgaca gccacctgtg cccacccaga atcactgaag 1320

ggtcagagca ttttctctgt gccaccagag agtttcgtgt gcgatgactt cctgaagcca 1380

cagatcatca cccagccaga aaccaccatg gctatggtgg gcaaggacat ccggtttaca 1440

tgctcagcag ccagcagcag cagctccccc atgacctttg cctggaagaa agacaatgaa 1500

gtcctgacca atgcagacat ggagaacttt gtccacgtcc acgcgcagga cggggaagtg 1560

atggagtaca ccaccatcct gcacctccgt caggtcactt tcgggcacga gggccgctac 1620

caatgtgtca tcaccaacca ctttggctcc acctattcac ataaggccag gctcaccgtg 1680

aatgtgttgc catcattcac caaaacgccc cacgacataa ccatccggac caccaccgtg 1740

gcccgcctcg aatgtgctgc cacaggtcac ccaaaccctc agattgcctg gcagaaggat 1800

ggaggcacgg atttccccgc tgcccgtgag cgacgcatgc atgtcatgcc ggatgacgac 1860

gtgtttttca tcactgatgt gaaaatagat gacgcagggg tttacagctg tactgctcag 1920

aactcagccg gttctatttc agctaatgcc accctgactg tcctagagac cccatccttg 1980

gtggtcccct tggaagaccg tgtggtatct gtgggagaaa cagtggccct ccaatgcaaa 2040

gccacgggga accctccgcc ccgcatcacc tggttcaagg gggaccgccc gctgagcctc 2100

actgagcggc accacctgac ccctgacaac cagctcctgg tggttcagaa cgtggtggca 2160

gaggatgcgg gccgatatac ctgtgagatg tccaacaccc tgggcacgga gcgagctcac 2220

agccagctga gcgtcctgcc cgcagcaggc tgcaggaagg atgggaccac ggtaggcatc 2280

ttcaccattg ctgtcgtgag cagcatcgtc ctgacgtcac tggtctgggt gtgcatcatc 2340

taccagacca ggaagaagag tgaagagtac agtgtcacca acacagatga aaccgtc 2397

<210>    3
<211>    761
<212>    PRT
<213>    Mus musculus

<400>    3
Gln Ala Gly Pro Arg Ala Pro Cys Ala Ala Ala Cys Thr Cys Ala Gly
1               5                   10                  15

Asp Ser Leu Asp Cys Ser Gly Arg Gly Leu Ala Thr Leu Pro Arg Asp
            20                  25                  30

Leu Pro Ser Trp Thr Arg Ser Leu Asn Leu Ser Tyr Asn Arg Leu Ser
        35                  40                  45

Glu Ile Asp Ser Ala Ala Phe Glu Asp Leu Thr Asn Leu Gln Glu Val
    50                  55                  60

Tyr Leu Asn Ser Asn Glu Leu Thr Ala Ile Pro Ser Leu Gly Ala Ala
65                  70                  75                  80

Ser Ile Gly Val Val Ser Leu Phe Leu Gln His Asn Lys Ile Leu Ser
                85                  90                  95

Val Asp Gly Ser Gln Leu Lys Ser Tyr Leu Ser Leu Glu Val Leu Asp
            100                 105                 110

Leu Ser Ser Asn Asn Ile Thr Glu Ile Arg Ser Ser Cys Phe Pro Asn
        115                 120                 125

Gly Leu Arg Ile Arg Glu Leu Asn Leu Ala Ser Asn Arg Ile Ser Ile
    130                 135                 140

Leu Glu Ser Gly Ala Phe Asp Gly Leu Ser Arg Ser Leu Leu Thr Leu
145                 150                 155                 160

Arg Leu Ser Lys Asn Arg Ile Thr Gln Leu Pro Val Lys Ala Phe Lys
                165                 170                 175

Leu Pro Arg Leu Thr Gln Leu Asp Leu Asn Arg Asn Arg Ile Arg Leu
            180                 185                 190

Ile Glu Gly Leu Thr Phe Gln Gly Leu Asp Ser Leu Glu Val Leu Arg
        195                 200                 205

Leu Gln Arg Asn Asn Ile Ser Arg Leu Thr Asp Gly Ala Phe Trp Gly
    210                 215                 220

Leu Ser Lys Met His Val Leu His Leu Glu Tyr Asn Ser Leu Val Glu
225                 230                 235                 240

Val Asn Ser Gly Ser Leu Tyr Gly Leu Thr Ala Leu His Gln Leu His
                245                 250                 255

Leu Ser Asn Asn Ser Ile Ser Arg Ile Gln Arg Asp Gly Trp Ser Phe
            260                 265                 270

Cys Gln Lys Leu His Glu Leu Ile Leu Ser Phe Asn Asn Leu Thr Arg
        275                 280                 285

Leu Asp Glu Glu Ser Leu Ala Glu Leu Ser Ser Leu Ser Ile Leu Arg
    290                 295                 300

Leu Ser His Asn Ala Ile Ser His Ile Ala Glu Gly Ala Phe Lys Gly
305                 310                 315                 320

Leu Lys Ser Leu Arg Val Leu Asp Leu Asp His Asn Glu Ile Ser Gly
                325                 330                 335

Thr Ile Glu Asp Thr Ser Gly Ala Phe Thr Gly Leu Asp Asn Leu Ser
            340                 345                 350

Lys Leu Thr Leu Phe Gly Asn Lys Ile Lys Ser Val Ala Lys Arg Ala
        355                 360                 365

Phe Ser Gly Leu Glu Ser Leu Glu His Leu Asn Leu Gly Glu Asn Ala
    370                 375                 380

Ile Arg Ser Val Gln Phe Asp Ala Phe Ala Lys Met Lys Asn Leu Lys
385                 390                 395                 400

Glu Leu Tyr Ile Ser Ser Glu Ser Phe Leu Cys Asp Cys Gln Leu Lys
                405                 410                 415

Trp Leu Pro Pro Trp Leu Met Gly Arg Met Leu Gln Ala Phe Val Thr
            420                 425                 430

Ala Thr Cys Ala His Pro Glu Ser Leu Lys Gly Gln Ser Ile Phe Ser
        435                 440                 445

Val Leu Pro Asp Ser Phe Val Cys Asp Asp Phe Pro Lys Pro Gln Ile
    450                 455                 460

Ile Thr Gln Pro Glu Thr Thr Met Ala Val Val Gly Lys Asp Ile Arg
465                 470                 475                 480

Phe Thr Cys Ser Ala Ala Ser Ser Ser Ser Ser Pro Met Thr Phe Ala
                485                 490                 495

Trp Lys Lys Asp Asn Glu Val Leu Ala Asn Ala Asp Met Glu Asn Phe
            500                 505                 510

Ala His Val Arg Ala Gln Asp Gly Glu Val Met Glu Tyr Thr Thr Ile
        515                 520                 525

Leu His Leu Arg His Val Thr Phe Gly His Glu Gly Arg Tyr Gln Cys
    530                 535                 540

Ile Ile Thr Asn His Phe Gly Ser Thr Tyr Ser His Lys Ala Arg Leu
545                 550                 555                 560

Thr Val Asn Val Leu Pro Ser Phe Thr Lys Ile Pro His Asp Ile Ala
                565                 570                 575

Ile Arg Thr Gly Thr Thr Ala Arg Leu Glu Cys Ala Ala Thr Gly His
            580                 585                 590

Pro Asn Pro Gln Ile Ala Trp Gln Lys Asp Gly Gly Thr Asp Phe Pro
        595                 600                 605

Ala Ala Arg Glu Arg Arg Met His Val Met Pro Asp Asp Asp Val Phe
    610                 615                 620

Phe Ile Thr Asp Val Lys Ile Asp Asp Met Gly Val Tyr Ser Cys Thr
625                 630                 635                 640

Ala Gln Asn Ser Ala Gly Ser Val Ser Ala Asn Ala Thr Leu Thr Val
                645                 650                 655

Leu Glu Thr Pro Ser Leu Ala Val Pro Leu Glu Asp Arg Val Val Thr
            660                 665                 670

Val Gly Glu Thr Val Ala Phe Gln Cys Lys Ala Thr Gly Ser Pro Thr
        675                 680                 685

Pro Arg Ile Thr Trp Leu Lys Gly Gly Arg Pro Leu Ser Leu Thr Glu
    690                 695                 700

Arg His His Phe Thr Pro Gly Asn Gln Leu Leu Val Val Gln Asn Val
705                 710                 715                 720

Met Ile Asp Asp Ala Gly Arg Tyr Thr Cys Glu Met Ser Asn Pro Leu
                725                 730                 735

Gly Thr Glu Arg Ala His Ser Gln Leu Ser Ile Leu Pro Thr Pro Gly
            740                 745                 750

Cys Arg Lys Asp Gly Thr Thr Val Gly
        755                 760

<210>    4
<211>    2283
<212>    DNA
<213>    Mus musculus

<400>    4
caggctggcc cgcgggcccc ctgcgcggcc gcctgcactt gcgccgggga ctcgctggac      60

tgcagtgggc gcgggctggc gacgctgccc cgggacctgc cctcctggac gcgcagccta     120

aacctgagtt ataacagact ctccgagatc gactctgctg cttttgagga cttgacgaat     180

ctgcaggaag tgtacctcaa cagcaatgag ctgacagcca taccatcact gggcgctgct     240

tccataggag ttgtctctct ctttttgcag cacaacaaga tccttagtgt ggatgggagc     300

cagctgaagt cgtacctgtc cttggaagtg ctggatctga gttccaacaa catcacggaa     360

attcggagct cctgtttccc gaacggcctg cgtataaggg aactcaactt ggcgagcaac     420

cgcatcagca tcctggagtc tggagcattt gatggtctgt cgcggtcact gctgactctc     480

cgtctgagca aaaacaggat cacccagctt cctgtgaaag cgttcaagct acccaggctg     540

acacaactag acctgaatcg gaatcggatt cggctgattg aaggcctcac gttccagggg     600

ctcgacagct tagaggtgct gaggcttcag aggaacaaca tcagcaggct gacggacggg     660

gccttctggg ggctgtctaa gatgcacgtg ctgcacctgg agtacaacag tctggtggaa     720

gtgaacagtg gctccctcta tggcctcaca gccctgcacc agctgcacct cagcaacaac     780

tccatctctc gaattcagcg tgatggctgg agcttctgcc aaaagctgca tgagttgatt     840

ctgtccttca acaacctcac gcggctggat gaggagagtc tagcggagtt gagcagcctc     900

agtatcctgc gcctcagtca caacgccatc agtcacattg ctgaaggcgc cttcaaggga     960

ctcaagagtc tgcgggtctt ggacctggac cataacgaga tctcgggtac aatcgaggat 1020

accagtggtg cctttacggg gcttgacaac ctcagcaagc tgactctgtt tggaaacaag 1080

atcaaatctg tggctaagag agccttctcg ggcctggaaa gcctggaaca cctgaacctt 1140

ggagagaatg caatcaggtc tgtccagttt gatgcctttg caaagatgaa gaaccttaaa 1200

gagctctaca tcagcagtga gagcttcctg tgtgactgcc agctcaagtg gctgccccca 1260

tggctaatgg gtaggatgct gcaggccttt gtgacagcca cctgtgccca tccagagtcg 1320

ctgaagggcc agagcatttt ctcagtgctg ccagacagct ttgtgtgtga tgactttcca 1380

aagccacaga tcatcaccca gcctgagacg accatggctg tggtgggcaa ggacatccgt 1440

ttcacatgct ccgcagccag cagcagcagc tcaccaatga ccttcgcctg gaagaaggac 1500

aatgaggtcc tggccaatgc agacatggag aactttgccc acgtccgtgc acaggacggc 1560

gaagtgatgg agtataccac tatcctgcac ctccgtcacg tcacctttgg gcacgagggc 1620

cgctaccagt gtatcatcac aaaccacttt ggctccacat actcccacaa agccaggctc 1680

actgtgaatg tgttgccatc attcactaaa ataccccatg acattgccat ccggactggc 1740

accacagccc gcctcgagtg tgctgccacg ggccacccta accctcagat tgcctggcag 1800

aaggatggag gcaccgattt cccggcagct cgtgagcgac gcatgcatgt tatgccagac 1860

gatgatgtgt tcttcatcac tgatgtgaaa atagacgaca tgggggtcta cagctgcact 1920

gcccagaact cggcaggctc ggtttcagcc aacgctaccc tcacagtctt agaaactcca 1980

tccttggcag tgcctctgga agaccgtgtg gtaactgtgg gagaaacagt ggccttccag 2040

tgcaaagcaa ccgggagccc cacaccacgc atcacctggc ttaagggagg tcgcccattg 2100

agcctcacag agcgccacca tttcactcca ggcaaccagc tgctggttgt tcagaatgtg 2160

atgatagacg atgcagggcg gtatacctgt gagatgtcta atcccctggg cactgagcga 2220

gcacatagcc agctgagcat tttacctacc cctggctgcc ggaaggatgg gaccaccgta 2280

ggc 2283

<210> 5
<211> 8
<212> PRT
<213> Artificial Sequence

<220>
<223> GTC110-01 heavy chain CDR1

<400> 5
Gly Phe Thr Phe Ser Asp Tyr Asp
1               5

<210> 6
<211> 9

<212>    PRT
<213>    Artificial Sequence

<220>
<223>    GTC110-01 heavy chain CDR2


<400>    6
Trp Ile Ser His Gly Gly Gly Ser Ile
1               5


<210>    7
<211>    20
<212>    PRT
<213>    Artificial Sequence

<220>
<223>    GTC110-01 heavy chain CDR3


<400>    7
Ala Arg Gly Leu Gly Leu Cys Lys Thr Gly Leu Cys Tyr Tyr Tyr Asp
1               5                   10                  15

Ala Met Asp Val
            20


<210>    8
<211>    8
<212>    PRT
<213>    Artificial Sequence

<220>
<223>    GTC110-01 light chain CDR1


<400>    8
Ser Ser Asn Ile Gly Asn Asn Ser
1               5


<210>    9
<211>    3
<212>    PRT
<213>    Artificial Sequence

<220>
<223>    GTC110-01 light chain CDR2


<400>    9
Ala Asp Asn
1


<210>    10
<211>    11
<212>    PRT
<213>    Artificial Sequence

<220>

<223>    GTC110-01 light chain CDR3

<400>    10
Ala Ala Trp Asp Ser Ser Leu Ser Ala Tyr Val
1               5                   10

<210>    11
<211>    127
<212>    PRT
<213>    Artificial Sequence

<220>
<223>    GTC110-01 heavy chain_variable region

<400>    11
Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Asp Tyr
            20                  25                  30

Asp Met Ser Trp Val Arg Gln Val Pro Gly Lys Gly Leu Glu Trp Val
        35                  40                  45

Ser Trp Ile Ser His Gly Gly Gly Ser Ile Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Gly Leu Gly Leu Cys Lys Thr Gly Leu Cys Tyr Tyr Tyr Asp
            100                 105                 110

Ala Met Asp Val Trp Gly Gln Gly Thr Leu Val Thr Val Ser Ser
        115                 120                 125

<210>    12
<211>    110
<212>    PRT
<213>    Artificial Sequence

<220>
<223>    GTC110-01 light chain_variable region

<400>    12
Gln Ser Val Leu Thr Gln Pro Pro Ser Ala Ser Gly Thr Pro Gly Gln
1               5                   10                  15

Arg Val Thr Ile Ser Cys Thr Gly Ser Ser Ser Asn Ile Gly Asn Asn
            20                  25                  30

Ser Val Thr Trp Tyr Gln Gln Leu Pro Gly Thr Ala Pro Lys Leu Leu
        35                  40                  45

Ile Tyr Ala Asp Asn Asn Arg Pro Ser Gly Val Pro Asp Arg Phe Ser
    50                  55                  60

Gly Ser Lys Ser Gly Thr Ser Ala Ser Leu Ala Ile Ser Gly Leu Arg
65                  70                  75                  80

Ser Glu Asp Glu Ala Asp Tyr Tyr Cys Ala Ala Trp Asp Ser Ser Leu
                85                  90                  95

Ser Ala Tyr Val Phe Gly Gly Gly Thr Lys Leu Thr Val Leu
            100                 105                 110


<210>    13
<211>    8
<212>    PRT
<213>    Artificial Sequence

<220>
<223>    GTC110-02 heavy chain CDR1


<400>    13
Gly Phe Thr Phe Ser Asp Tyr Tyr
1               5


<210>    14
<211>    9
<212>    PRT
<213>    Artificial Sequence

<220>
<223>    GTC110-02 heavy chain CDR2


<400>    14
Gly Ile Ser His Asp Ser Gly Ser Lys
1               5


<210>    15
<211>    9
<212>    PRT
<213>    Artificial Sequence

<220>
<223>    GTC110-02 heavy chain CDR3


<400>    15
Ala Arg His Trp Thr Thr Phe Asp Tyr
1               5


<210>    16
<211>    8
<212>    PRT
<213>    Artificial Sequence

<220>
<223>    GTC110-02 light chain CDR1

<400>    16
Ser Ser Asn Ile Gly Ser Asn Asn
1               5

<210>    17
<211>    3
<212>    PRT
<213>    Artificial Sequence

<220>
<223>    GTC110-02 light chain CDR2

<400>    17
Ala Asn Ser
1

<210>    18
<211>    11
<212>    PRT
<213>    Artificial Sequence

<220>
<223>    GTC110-02 light chain CDR3

<400>    18
Gly Ala Trp Asp Tyr Ser Leu Ser Ala Tyr Val
1               5                   10

<210>    19
<211>    116
<212>    PRT
<213>    Artificial Sequence

<220>
<223>    GTC110-02 heavy chain_variable region

<400>    19
Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Asp Tyr
            20                  25                  30

Tyr Met Ser Trp Val Arg Gln Ala Pro Gly Lys Gly Leu Glu Trp Val
        35                  40                  45

Ser Gly Ile Ser His Asp Ser Gly Ser Lys Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg His Trp Thr Thr Phe Asp Tyr Trp Gly Gln Gly Thr Leu Val
            100                 105                 110

Thr Val Ser Ser
        115

<210> 20
<211> 110
<212> PRT
<213> Artificial Sequence

<220>
<223> GTC110-02 light chain_variable region

<400> 20
Gln Ser Val Leu Thr Gln Pro Pro Ser Ala Ser Gly Thr Pro Gly Gln
1               5                   10                  15

Arg Val Thr Ile Ser Cys Ser Gly Ser Ser Ser Asn Ile Gly Ser Asn
            20                  25                  30

Asn Val Thr Trp Tyr Gln Gln Leu Pro Gly Thr Ala Pro Lys Leu Leu
        35                  40                  45

Ile Tyr Ala Asn Ser Asn Arg Pro Ser Gly Val Pro Asp Arg Phe Ser
    50                  55                  60

Gly Ser Lys Ser Gly Thr Ser Ala Ser Leu Ala Ile Ser Gly Leu Arg
65                  70                  75                  80

Ser Glu Asp Glu Ala Asp Tyr Tyr Cys Gly Ala Trp Asp Tyr Ser Leu
                85                  90                  95

Ser Ala Tyr Val Phe Gly Gly Gly Thr Lys Leu Thr Val Leu
            100                 105                 110

<210> 21
<211> 8
<212> PRT
<213> Artificial Sequence

<220>
<223> GTC110-03 heavy chain CDR1

<400> 21
Gly Phe Thr Phe Ser Asn Tyr Ala
1               5

<210> 22
<211> 9
<212> PRT
<213> Artificial Sequence

<220>
<223> GTC110-03 heavy chain CDR2

<400> 22

Ala Ile Tyr Pro Gly Gly Gly Ser Ile
1               5

<210>    23
<211>    20
<212>    PRT
<213>    Artificial Sequence

<220>
<223>    GTC110-03 heavy chain CDR3

<400>    23
Ala Arg Asp Ile Leu Pro Cys Pro Trp Gly Arg Cys Tyr Tyr Asp Tyr
1               5                   10                  15

Ala Met Asp Val
            20

<210>    24
<211>    8
<212>    PRT
<213>    Artificial Sequence

<220>
<223>    GTC110-03 light chain CDR1

<400>    24
Ser Ser Asn Ile Gly Ser Asn Thr
1               5

<210>    25
<211>    3
<212>    PRT
<213>    Artificial Sequence

<220>
<223>    GTC110-03 light chain CDR2

<400>    25
Ala Asp Asn
1

<210>    26
<211>    11
<212>    PRT
<213>    Artificial Sequence

<220>
<223>    GTC110-03 light chain CDR3

<400>    26
Gly Thr Trp Asp Tyr Ser Leu Ser Gly Tyr Val
1               5                   10

<210>    27
<211>    127
<212>    PRT
<213>    Artificial Sequence

<220>
<223>    GTC110-03 heavy chain_variable region


<400>    27
Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Asn Tyr
            20                  25                  30

Ala Met Ser Trp Val Arg Gln Ala Pro Gly Lys Gly Leu Glu Trp Val
        35                  40                  45

Ser Ala Ile Tyr Pro Gly Gly Gly Ser Ile Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Asp Ile Leu Pro Cys Pro Trp Gly Arg Cys Tyr Tyr Asp Tyr
            100                 105                 110

Ala Met Asp Val Trp Gly Gln Gly Thr Leu Val Thr Val Ser Ser
        115                 120                 125




<210>    28
<211>    110
<212>    PRT
<213>    Artificial Sequence

<220>
<223>    GTC110-03 light chain_variable region


<400>    28
Gln Ser Val Leu Thr Gln Pro Pro Ser Ala Ser Gly Thr Pro Gly Gln
1               5                   10                  15

Arg Val Thr Ile Ser Cys Ser Asp Ser Ser Ser Asn Ile Gly Ser Asn
            20                  25                  30

Thr Val Ser Trp Tyr Gln Gln Leu Pro Gly Thr Ala Pro Lys Leu Leu
        35                  40                  45

Ile Tyr Ala Asp Asn Asn Arg Pro Ser Gly Val Pro Asp Arg Phe Ser
    50                  55                  60

Gly Ser Lys Ser Gly Thr Ser Ala Ser Leu Ala Ile Ser Gly Leu Arg
65                  70                  75                  80

Ser Glu Asp Glu Ala Asp Tyr Tyr Cys Gly Thr Trp Asp Tyr Ser Leu

85                  90                  95

Ser Gly Tyr Val Phe Gly Gly Gly Thr Lys Leu Thr Val Leu
            100                 105                 110

<210>    29
<211>    8
<212>    PRT
<213>    Artificial Sequence

<220>
<223>    GTC110-04 heavy chain CDR1

<400>    29
Gly Phe Thr Phe Ser Asn Tyr Ala
1               5

<210>    30
<211>    9
<212>    PRT
<213>    Artificial Sequence

<220>
<223>    GTC110-04 heavy chain CDR2

<400>    30
Val Ile Ser His Gly Gly Gly Ser Thr
1               5

<210>    31
<211>    20
<212>    PRT
<213>    Artificial Sequence

<220>
<223>    GTC110-04 heavy chain CDR3

<400>    31
Ala Arg Val Ile Ser Asn Cys His Leu Gly Val Cys Tyr Tyr Ser Asn
1               5                   10                  15

Gly Met Asp Val
            20

<210>    32
<211>    8
<212>    PRT
<213>    Artificial Sequence

<220>
<223>    GTC110-04 light chain CDR1

<400>    32
Ser Ser Asn Ile Gly Asn Asn Asp
1               5

<210>    33
<211>    3
<212>    PRT
<213>    Artificial Sequence

<220>
<223>    GTC110-04 light chain CDR2


<400>    33
Ser Asp Ser
1


<210>    34
<211>    11
<212>    PRT
<213>    Artificial Sequence

<220>
<223>    GTC110-04 light chain CDR3


<400>    34
Gly Thr Trp Asp Tyr Ser Leu Ser Gly Tyr Val
1               5                   10


<210>    35
<211>    127
<212>    PRT
<213>    Artificial Sequence

<220>
<223>    GTC110-04 heavy chain_variable region


<400>    35
Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Asn Tyr
            20                  25                  30

Ala Met Ser Trp Val Arg Gln Ala Pro Gly Lys Gly Leu Glu Trp Val
        35                  40                  45

Ser Val Ile Ser His Gly Gly Gly Ser Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Val Ile Ser Asn Cys His Leu Gly Val Cys Tyr Tyr Ser Asn
            100                 105                 110

Gly Met Asp Val Trp Gly Gln Gly Thr Leu Val Thr Val Ser Ser
        115                 120                 125

<210>    36
<211>    110
<212>    PRT
<213>    Artificial Sequence

<220>
<223>    GTC110-04 light chain_variable region


<400>    36
Gln Ser Val Leu Thr Gln Pro Pro Ser Ala Ser Gly Thr Pro Gly Gln
1               5                   10                  15

Arg Val Thr Ile Ser Cys Ser Gly Ser Ser Ser Asn Ile Gly Asn Asn
            20                  25                  30

Asp Val Tyr Trp Tyr Gln Gln Leu Pro Gly Thr Ala Pro Lys Leu Leu
        35                  40                  45

Ile Tyr Ser Asp Ser Gln Arg Pro Ser Gly Val Pro Asp Arg Phe Ser
    50                  55                  60

Gly Ser Lys Ser Gly Thr Ser Ala Ser Leu Ala Ile Ser Gly Leu Arg
65                  70                  75                  80

Ser Glu Asp Glu Ala Asp Tyr Tyr Cys Gly Thr Trp Asp Tyr Ser Leu
                85                  90                  95

Ser Gly Tyr Val Phe Gly Gly Gly Thr Lys Leu Thr Val Leu
            100                 105                 110


<210>    37
<211>    328
<212>    PRT
<213>    Mus musculus

<400>    37
Thr Thr Ala Pro Ser Val Tyr Pro Leu Ala Pro Val Cys Gly Asp Thr
1               5                   10                  15

Thr Gly Ser Ser Val Thr Leu Gly Cys Leu Val Lys Gly Tyr Phe Pro
            20                  25                  30

Glu Pro Val Thr Leu Thr Trp Asn Ser Gly Ser Leu Ser Ser Gly Val
        35                  40                  45

His Thr Phe Pro Ala Val Leu Gln Ser Asp Leu Tyr Thr Leu Ser Ser
    50                  55                  60

Ser Val Thr Val Thr Ser Ser Thr Trp Pro Ser Gln Ser Ile Thr Cys
65                  70                  75                  80

Asn Val Ala His Pro Ala Ser Ser Thr Lys Val Asp Lys Lys Ile Glu
                85                  90                  95

Pro Arg Gly Pro Thr Ile Lys Pro Cys Pro Pro Cys Lys Cys Pro Ala
            100                 105                 110

Pro Asn Leu Leu Gly Gly Pro Ser Val Phe Ile Phe Pro Pro Lys Ile
        115                 120                 125

Lys Asp Val Leu Met Ile Ser Leu Ser Pro Ile Val Thr Cys Val Val
    130                 135                 140

Val Asp Val Ser Glu Asp Asp Pro Asp Val Gln Ile Ser Trp Phe Val
145                 150                 155                 160

Asn Asn Val Glu Val His Thr Ala Gln Thr Gln Thr His Arg Glu Asp
                165                 170                 175

Tyr Asn Ser Thr Leu Arg Val Val Ser Ala Leu Pro Ile Gln His Gln
            180                 185                 190

Asp Trp Met Ser Gly Lys Glu Phe Lys Cys Lys Val Asn Asn Lys Asp
        195                 200                 205

Leu Pro Ala Pro Ile Glu Arg Thr Ile Ser Lys Pro Lys Gly Ser Val
    210                 215                 220

Arg Ala Pro Gln Val Tyr Val Leu Pro Pro Pro Glu Glu Glu Met Thr
225                 230                 235                 240

Lys Lys Gln Val Thr Leu Thr Cys Met Val Thr Asp Phe Met Pro Glu
                245                 250                 255

Asp Ile Tyr Val Glu Trp Thr Asn Asn Gly Lys Thr Glu Leu Asn Tyr
            260                 265                 270

Lys Asn Thr Glu Pro Val Leu Asp Ser Asp Gly Ser Tyr Phe Met Tyr
        275                 280                 285

Ser Lys Leu Arg Val Glu Lys Lys Asn Trp Val Glu Arg Asn Ser Tyr
    290                 295                 300

Ser Cys Ser Val Val His Glu Gly Leu His Asn His His Thr Thr Lys
305                 310                 315                 320

Ser Phe Ser Arg Thr Pro Gly Lys
                325

<210>    38
<211>    107
<212>    PRT
<213>    Mus musculus

<400>    38
Arg Thr Val Ala Ala Pro Thr Val Ser Ile Phe Pro Pro Ser Ser Glu
 1               5                  10                  15

Gln Leu Thr Ser Gly Gly Ala Ser Val Val Cys Phe Leu Asn Asn Phe
            20                  25                  30

Tyr Pro Lys Asp Ile Asn Val Lys Trp Lys Ile Asp Gly Ser Glu Arg
        35                  40                  45

Gln Asn Gly Val Leu Asn Ser Trp Thr Asp Gln Asp Ser Lys Asp Ser
    50                  55                  60

Thr Tyr Ser Met Ser Ser Thr Leu Thr Leu Thr Lys Asp Glu Tyr Glu
65                  70                  75                  80

Arg His Asn Ser Tyr Thr Cys Glu Ala Thr His Lys Thr Ser Thr Ser
                85                  90                  95

Pro Ile Val Lys Ser Phe Asn Arg Asn Glu Cys
            100                 105

<210>   39
<211>   330
<212>   PRT
<213>   Homo sapiens

<400>   39


<210>    40
<211>    330
<212>    PRT
<213>    Homo sapiens

<400>    40
Ala Ser Thr Lys Gly Pro Ser Val Phe Pro Leu Ala Pro Ser Ser Lys
 1               5                  10                  15

Ser Thr Ser Gly Gly Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Ser Leu Gly Thr Gln Thr
65                  70                  75                  80

Tyr Ile Cys Asn Val Asn His Lys Pro Ser Asn Thr Lys Val Asp Lys
                85                  90                  95

Lys Val Glu Pro Lys Ser Cys Asp Lys Thr His Thr Cys Pro Pro Cys
            100                 105                 110

Pro Ala Pro Glu Leu Leu Gly Gly Pro Ser Val Phe Leu Phe Pro Pro
        115                 120                 125

Lys Pro Lys Asp Thr Leu Met Ile Ser Arg Thr Pro Glu Val Thr Cys
    130                 135                 140

Val Val Val Asp Val Ser His Glu Asp Pro Glu Val Lys Phe Asn Trp
145                 150                 155                 160

Tyr Val Asp Gly Val Glu Val His Asn Ala Lys Thr Lys Pro Arg Glu
                165                 170                 175

Glu Gln Tyr Asn Ser Thr Tyr Arg Val Val Ser Val Leu Thr Val Leu
            180                 185                 190

His Gln Asp Trp Leu Asn Gly Lys Glu Tyr Lys Cys Lys Val Ser Asn
        195                 200                 205

Lys Ala Leu Pro Ala Pro Ile Glu Lys Thr Ile Ser Lys Ala Lys Gly
    210                 215                 220

Gln Pro Arg Glu Pro Gln Val Tyr Thr Leu Pro Pro Ser Arg Glu Glu
225                 230                 235                 240

Met Thr Lys Asn Gln Val Ser Leu Thr Cys Leu Val Lys Gly Phe Tyr
                245                 250                 255

Pro Ser Asp Ile Ala Val Glu Trp Glu Ser Asn Gly Gln Pro Glu Asn
            260                 265                 270

Asn Tyr Lys Thr Thr Pro Pro Val Leu Asp Ser Asp Gly Ser Phe Phe
        275                 280                 285

Leu Tyr Ser Lys Leu Thr Val Asp Lys Ser Arg Trp Gln Gln Gly Asn
    290                 295                 300

Val Phe Ser Cys Ser Val Met His Glu Ala Leu His Asn His Tyr Thr
305                 310                 315                 320

Gln Lys Ser Leu Ser Leu Ser Pro Gly Lys
                325                 330


<210>    41
<211>    107
<212>    PRT
<213>    Homo sapiens

<400>    41


<210>    42
<211>    326
<212>    PRT
<213>    Homo sapiens

<400>    42
Ala Ser Thr Lys Gly Pro Ser Val Phe Pro Leu Ala Pro Cys Ser Arg
 1               5                  10                  15

Ser Thr Ser Glu Ser Thr Ala Ala Leu Gly Cys Leu Val Lys Asp Tyr
            20                  25                  30

Phe Pro Glu Pro Val Thr Val Ser Trp Asn Ser Gly Ala Leu Thr Ser

35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Asn Phe Gly Thr Gln Thr
65                  70                  75                  80

Tyr Thr Cys Asn Val Asp His Lys Pro Ser Asn Thr Lys Val Asp Lys
                85                  90                  95

Thr Val Glu Arg Lys Cys Cys Val Glu Cys Pro Pro Cys Pro Ala Pro
            100                 105                 110

Pro Val Ala Gly Pro Ser Val Phe Leu Phe Pro Pro Lys Pro Lys Asp
        115                 120                 125

Thr Leu Met Ile Ser Arg Thr Pro Glu Val Thr Cys Val Val Val Asp
    130                 135                 140

Val Ser His Glu Asp Pro Glu Val Gln Phe Asn Trp Tyr Val Asp Gly
145                 150                 155                 160

Val Glu Val His Asn Ala Lys Thr Lys Pro Arg Glu Glu Gln Phe Asn
                165                 170                 175

Ser Thr Phe Arg Val Val Ser Val Leu Thr Val Val His Gln Asp Trp
            180                 185                 190

Leu Asn Gly Lys Glu Tyr Lys Cys Lys Val Ser Asn Lys Gly Leu Pro
        195                 200                 205

Ala Pro Ile Glu Lys Thr Ile Ser Lys Thr Lys Gly Gln Pro Arg Glu
    210                 215                 220

Pro Gln Val Tyr Thr Leu Pro Pro Ser Arg Glu Glu Met Thr Lys Asn
225                 230                 235                 240

Gln Val Ser Leu Thr Cys Leu Val Lys Gly Phe Tyr Pro Ser Asp Ile
                245                 250                 255

Ser Val Glu Trp Glu Ser Asn Gly Gln Pro Glu Asn Asn Tyr Lys Thr
            260                 265                 270

Thr Pro Pro Met Leu Asp Ser Asp Gly Ser Phe Phe Leu Tyr Ser Lys
        275                 280                 285

Leu Thr Val Asp Lys Ser Arg Trp Gln Gln Gly Asn Val Phe Ser Cys
    290                 295                 300

Ser Val Met His Glu Ala Leu His Asn His Tyr Thr Gln Lys Ser Leu
305                 310                 315                 320

Ser Leu Ser Pro Gly Lys
                325


<210>   43
<211>   377
<212>   PRT
<213>   Homo sapiens

<400>   43

Ala Ser Thr Lys Gly Pro Ser Val Phe Pro Leu Ala Pro Cys Ser Arg
1               5                   10                  15

Ser Thr Ser Gly Gly Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Ser Leu Gly Thr Gln Thr
65                  70                  75                  80

Tyr Thr Cys Asn Val Asn His Lys Pro Ser Asn Thr Lys Val Asp Lys
                85                  90                  95

Arg Val Glu Leu Lys Thr Pro Leu Gly Asp Thr Thr His Thr Cys Pro
            100                 105                 110

Arg Cys Pro Glu Pro Lys Ser Cys Asp Thr Pro Pro Pro Cys Pro Arg
        115                 120                 125

Cys Pro Glu Pro Lys Ser Cys Asp Thr Pro Pro Pro Cys Pro Arg Cys
    130                 135                 140

Pro Glu Pro Lys Ser Cys Asp Thr Pro Pro Pro Cys Pro Arg Cys Pro
145                 150                 155                 160

Ala Pro Glu Leu Leu Gly Gly Pro Ser Val Phe Leu Phe Pro Pro Lys
                165                 170                 175

Pro Lys Asp Thr Leu Met Ile Ser Arg Thr Pro Glu Val Thr Cys Val
            180                 185                 190

Val Val Asp Val Ser His Glu Asp Pro Glu Val Gln Phe Lys Trp Tyr
        195                 200                 205

Val Asp Gly Val Glu Val His Asn Ala Lys Thr Lys Pro Arg Glu Glu
    210                 215                 220

Gln Tyr Asn Ser Thr Phe Arg Val Val Ser Val Leu Thr Val Leu His
225                 230                 235                 240

Gln Asp Trp Leu Asn Gly Lys Glu Tyr Lys Cys Lys Val Ser Asn Lys
                245                 250                 255

Ala Leu Pro Ala Pro Ile Glu Lys Thr Ile Ser Lys Thr Lys Gly Gln
            260                 265                 270

Pro Arg Glu Pro Gln Val Tyr Thr Leu Pro Pro Ser Arg Glu Glu Met
        275                 280                 285

Thr Lys Asn Gln Val Ser Leu Thr Cys Leu Val Lys Gly Phe Tyr Pro
    290                 295                 300

Ser Asp Ile Ala Val Glu Trp Glu Ser Ser Gly Gln Pro Glu Asn Asn
305                 310                 315                 320

Tyr Asn Thr Thr Pro Pro Met Leu Asp Ser Asp Gly Ser Phe Phe Leu
                325                 330                 335

Tyr Ser Lys Leu Thr Val Asp Lys Ser Arg Trp Gln Gln Gly Asn Ile
            340                 345                 350

Phe Ser Cys Ser Val Met His Glu Ala Leu His Asn Arg Phe Thr Gln
        355                 360                 365

Lys Ser Leu Ser Leu Ser Pro Gly Lys
    370                 375

<210>   44
<211>   327
<212>   PRT
<213>   Homo sapiens

<400>   44
Ala Ser Thr Lys Gly Pro Ser Val Phe Pro Leu Ala Pro Cys Ser Arg
1               5                   10                  15

Ser Thr Ser Glu Ser Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Ser Leu Gly Thr Lys Thr
65                  70                  75                  80

Tyr Thr Cys Asn Val Asp His Lys Pro Ser Asn Thr Lys Val Asp Lys
                85                  90                  95

Arg Val Glu Ser Lys Tyr Gly Pro Pro Cys Pro Ser Cys Pro Ala Pro
            100                 105                 110

Glu Phe Leu Gly Gly Pro Ser Val Phe Leu Phe Pro Pro Lys Pro Lys
        115                 120                 125

Asp Thr Leu Met Ile Ser Arg Thr Pro Glu Val Thr Cys Val Val Val
    130                 135                 140

Asp Val Ser Gln Glu Asp Pro Glu Val Gln Phe Asn Trp Tyr Val Asp
145                 150                 155                 160

Gly Val Glu Val His Asn Ala Lys Thr Lys Pro Arg Glu Glu Gln Phe
                165                 170                 175

Asn Ser Thr Tyr Arg Val Val Ser Val Leu Thr Val Leu His Gln Asp
            180                 185                 190

Trp Leu Asn Gly Lys Glu Tyr Lys Cys Lys Val Ser Asn Lys Gly Leu
        195                 200                 205

Pro Ser Ser Ile Glu Lys Thr Ile Ser Lys Ala Lys Gly Gln Pro Arg
    210                 215                 220

Glu Pro Gln Val Tyr Thr Leu Pro Pro Ser Gln Glu Glu Met Thr Lys
225                 230                 235                 240

Asn Gln Val Ser Leu Thr Cys Leu Val Lys Gly Phe Tyr Pro Ser Asp
                245                 250                 255

Ile Ala Val Glu Trp Glu Ser Asn Gly Gln Pro Glu Asn Asn Tyr Lys
            260                 265                 270

Thr Thr Pro Pro Val Leu Asp Ser Asp Gly Ser Phe Phe Leu Tyr Ser
        275                 280                 285

Arg Leu Thr Val Asp Lys Ser Arg Trp Gln Glu Gly Asn Val Phe Ser
    290                 295                 300

Cys Ser Val Met His Glu Ala Leu His Asn His Tyr Thr Gln Lys Ser
305                 310                 315                 320

Leu Ser Leu Ser Leu Gly Lys
                325

<210>    45
<211>    245
<212>    PRT
<213>    Artificial Sequence

<220>
<223>    Hybrid Fc_Heavy region

<400>    45
Arg Asn Thr Gly Arg Gly Gly Glu Glu Lys Lys Lys Glu Lys Glu Lys
 1               5                  10                  15

Glu Glu Gln Glu Glu Arg Glu Thr Lys Thr Pro Glu Cys Pro Ser His
            20                  25                  30

Thr Gln Pro Leu Gly Val Phe Leu Phe Pro Pro Lys Pro Lys Asp Thr
        35                  40                  45

Leu Met Ile Ser Arg Thr Pro Glu Val Thr Cys Val Val Val Asp Val
    50                  55                  60

Ser Gln Glu Asp Pro Glu Val Gln Phe Asn Trp Tyr Val Asp Gly Val
65                  70                  75                  80

Glu Val His Asn Ala Lys Thr Lys Pro Arg Glu Glu Gln Phe Asn Ser
                85                  90                  95

Thr Tyr Arg Val Val Ser Val Leu Thr Val Leu His Gln Asp Trp Leu
            100                 105                 110

Asn Gly Lys Glu Tyr Lys Cys Lys Val Ser Asn Lys Gly Leu Pro Ser
        115                 120                 125

Ser Ile Glu Lys Thr Ile Ser Lys Ala Lys Gly Gln Pro Arg Glu Pro
    130                 135                 140

Gln Val Tyr Thr Leu Pro Pro Ser Gln Glu Glu Met Thr Lys Asn Gln
145                 150                 155                 160

Val Ser Leu Thr Cys Leu Val Lys Gly Phe Tyr Pro Ser Asp Ile Ala
                165                 170                 175

Val Glu Trp Glu Ser Asn Gly Gln Pro Glu Asn Asn Tyr Lys Thr Thr
            180                 185                 190

Pro Pro Val Leu Asp Ser Asp Gly Ser Phe Phe Leu Tyr Ser Arg Leu
        195                 200                 205

Thr Val Asp Lys Ser Arg Trp Gln Glu Gly Asn Val Phe Ser Cys Ser
    210                 215                 220

Val Met His Glu Ala Leu His Asn His Tyr Thr Gln Lys Ser Leu Ser
225                 230                 235                 240

Leu Ser Leu Gly Lys
                245


<210>    46
<211>    457
<212>    PRT
<213>    Artificial Sequence

<220>
<223>    GTC110-01 heavy chain_mouse IgG2 Fc_full sequence


<400>    46
Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Asp Tyr
            20                  25                  30

Asp Met Ser Trp Val Arg Gln Val Pro Gly Lys Gly Leu Glu Trp Val
        35                  40                  45

Ser Trp Ile Ser His Gly Gly Gly Ser Ile Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Gly Leu Gly Leu Cys Lys Thr Gly Leu Cys Tyr Tyr Tyr Asp
            100                 105                 110

Ala Met Asp Val Trp Gly Gln Gly Thr Leu Val Thr Val Ser Ser Ala
        115                 120                 125

Ser Thr Thr Ala Pro Ser Val Tyr Pro Leu Ala Pro Val Cys Gly Asp
    130                 135                 140

Thr Thr Gly Ser Ser Val Thr Leu Gly Cys Leu Val Lys Gly Tyr Phe
145                 150                 155                 160

Pro Glu Pro Val Thr Leu Thr Trp Asn Ser Gly Ser Leu Ser Ser Gly
                165                 170                 175

Val His Thr Phe Pro Ala Val Leu Gln Ser Asp Leu Tyr Thr Leu Ser
            180                 185                 190

Ser Ser Val Thr Val Thr Ser Ser Thr Trp Pro Ser Gln Ser Ile Thr
        195                 200                 205

Cys Asn Val Ala His Pro Ala Ser Ser Thr Lys Val Asp Lys Lys Ile

210                 215                 220

Glu Pro Arg Gly Pro Thr Ile Lys Pro Cys Pro Pro Cys Lys Cys Pro
225                 230                 235                 240

Ala Pro Asn Leu Leu Gly Gly Pro Ser Val Phe Ile Phe Pro Pro Lys
                245                 250                 255

Ile Lys Asp Val Leu Met Ile Ser Leu Ser Pro Ile Val Thr Cys Val
            260                 265                 270

Val Val Asp Val Ser Glu Asp Asp Pro Asp Val Gln Ile Ser Trp Phe
        275                 280                 285

Val Asn Asn Val Glu Val His Thr Ala Gln Thr Gln Thr His Arg Glu
    290                 295                 300

Asp Tyr Asn Ser Thr Leu Arg Val Val Ser Ala Leu Pro Ile Gln His
305                 310                 315                 320

Gln Asp Trp Met Ser Gly Lys Glu Phe Lys Cys Lys Val Asn Asn Lys
                325                 330                 335

Asp Leu Pro Ala Pro Ile Glu Arg Thr Ile Ser Lys Pro Lys Gly Ser
            340                 345                 350

Val Arg Ala Pro Gln Val Tyr Val Leu Pro Pro Pro Glu Glu Glu Met
        355                 360                 365

Thr Lys Lys Gln Val Thr Leu Thr Cys Met Val Thr Asp Phe Met Pro
    370                 375                 380

Glu Asp Ile Tyr Val Glu Trp Thr Asn Asn Gly Lys Thr Glu Leu Asn
385                 390                 395                 400

Tyr Lys Asn Thr Glu Pro Val Leu Asp Ser Asp Gly Ser Tyr Phe Met
                405                 410                 415

Tyr Ser Lys Leu Arg Val Glu Lys Lys Asn Trp Val Glu Arg Asn Ser
            420                 425                 430

Tyr Ser Cys Ser Val Val His Glu Gly Leu His Asn His His Thr Thr
        435                 440                 445

Lys Ser Phe Ser Arg Thr Pro Gly Lys
    450                 455


<210>   47
<211>   217
<212>   PRT
<213>   Artificial Sequence

<220>
<223>   GTC110-01 light chain_mouse IgG2 Fc_full sequence


<400>   47
Gln Ser Val Leu Thr Gln Pro Pro Ser Ala Ser Gly Thr Pro Gly Gln
1               5                   10                  15

Arg Val Thr Ile Ser Cys Thr Gly Ser Ser Ser Asn Ile Gly Asn Asn
            20                  25                  30

Ser Val Thr Trp Tyr Gln Gln Leu Pro Gly Thr Ala Pro Lys Leu Leu
        35                  40                  45

Ile Tyr Ala Asp Asn Asn Arg Pro Ser Gly Val Pro Asp Arg Phe Ser
    50                  55                  60

Gly Ser Lys Ser Gly Thr Ser Ala Ser Leu Ala Ile Ser Gly Leu Arg
65                  70                  75                  80

Ser Glu Asp Glu Ala Asp Tyr Tyr Cys Ala Ala Trp Asp Ser Ser Leu
                85                  90                  95

Ser Ala Tyr Val Phe Gly Gly Gly Thr Lys Leu Thr Val Leu Arg Thr
            100                 105                 110

Val Ala Ala Pro Thr Val Ser Ile Phe Pro Pro Ser Ser Glu Gln Leu
        115                 120                 125

Thr Ser Gly Gly Ala Ser Val Val Cys Phe Leu Asn Asn Phe Tyr Pro
    130                 135                 140

Lys Asp Ile Asn Val Lys Trp Lys Ile Asp Gly Ser Glu Arg Gln Asn
145                 150                 155                 160

Gly Val Leu Asn Ser Trp Thr Asp Gln Asp Ser Lys Asp Ser Thr Tyr
                165                 170                 175

Ser Met Ser Ser Thr Leu Thr Leu Thr Lys Asp Glu Tyr Glu Arg His
            180                 185                 190

Asn Ser Tyr Thr Cys Glu Ala Thr His Lys Thr Ser Thr Ser Pro Ile
        195                 200                 205

Val Lys Ser Phe Asn Arg Asn Glu Cys
    210                 215


<210>   48
<211>   446
<212>   PRT
<213>   Artificial Sequence

<220>
<223>   GTC110-02 heavy chain_mouse IgG2 Fc_full sequence


<400>   48
Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Asp Tyr
            20                  25                  30

Tyr Met Ser Trp Val Arg Gln Ala Pro Gly Lys Gly Leu Glu Trp Val
        35                  40                  45

Ser Gly Ile Ser His Asp Ser Gly Ser Lys Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg His Trp Thr Thr Phe Asp Tyr Trp Gly Gln Gly Thr Leu Val
            100                 105                 110

Thr Val Ser Ser Ala Ser Thr Thr Ala Pro Ser Val Tyr Pro Leu Ala
        115                 120                 125

Pro Val Cys Gly Asp Thr Thr Gly Ser Ser Val Thr Leu Gly Cys Leu
    130                 135                 140

Val Lys Gly Tyr Phe Pro Glu Pro Val Thr Leu Thr Trp Asn Ser Gly
145                 150                 155                 160

Ser Leu Ser Ser Gly Val His Thr Phe Pro Ala Val Leu Gln Ser Asp
                165                 170                 175

Leu Tyr Thr Leu Ser Ser Ser Val Thr Val Thr Ser Ser Thr Trp Pro
            180                 185                 190

Ser Gln Ser Ile Thr Cys Asn Val Ala His Pro Ala Ser Ser Thr Lys
        195                 200                 205

Val Asp Lys Lys Ile Glu Pro Arg Gly Pro Thr Ile Lys Pro Cys Pro
    210                 215                 220

Pro Cys Lys Cys Pro Ala Pro Asn Leu Leu Gly Gly Pro Ser Val Phe
225                 230                 235                 240

Ile Phe Pro Pro Lys Ile Lys Asp Val Leu Met Ile Ser Leu Ser Pro
                245                 250                 255

Ile Val Thr Cys Val Val Val Asp Val Ser Glu Asp Asp Pro Asp Val
            260                 265                 270

Gln Ile Ser Trp Phe Val Asn Asn Val Glu Val His Thr Ala Gln Thr
        275                 280                 285

Gln Thr His Arg Glu Asp Tyr Asn Ser Thr Leu Arg Val Val Ser Ala
    290                 295                 300

Leu Pro Ile Gln His Gln Asp Trp Met Ser Gly Lys Glu Phe Lys Cys
305                 310                 315                 320

Lys Val Asn Asn Lys Asp Leu Pro Ala Pro Ile Glu Arg Thr Ile Ser
                325                 330                 335

Lys Pro Lys Gly Ser Val Arg Ala Pro Gln Val Tyr Val Leu Pro Pro
            340                 345                 350

Pro Glu Glu Glu Met Thr Lys Lys Gln Val Thr Leu Thr Cys Met Val
        355                 360                 365

Thr Asp Phe Met Pro Glu Asp Ile Tyr Val Glu Trp Thr Asn Asn Gly
    370                 375                 380

Lys Thr Glu Leu Asn Tyr Lys Asn Thr Glu Pro Val Leu Asp Ser Asp
385                 390                 395                 400

Gly Ser Tyr Phe Met Tyr Ser Lys Leu Arg Val Glu Lys Lys Asn Trp
                405                 410                 415

Val Glu Arg Asn Ser Tyr Ser Cys Ser Val Val His Glu Gly Leu His
                420                 425                 430

Asn His His Thr Thr Lys Ser Phe Ser Arg Thr Pro Gly Lys
        435                 440                 445

<210>    49
<211>    217
<212>    PRT
<213>    Artificial Sequence

<220>
<223>    GTC110-02 light chain_mouse IgG2 Fc_full sequence

<400>    49
Gln Ser Val Leu Thr Gln Pro Pro Ser Ala Ser Gly Thr Pro Gly Gln
1               5                   10                  15

Arg Val Thr Ile Ser Cys Ser Gly Ser Ser Ser Asn Ile Gly Ser Asn
            20                  25                  30

Asn Val Thr Trp Tyr Gln Gln Leu Pro Gly Thr Ala Pro Lys Leu Leu
        35                  40                  45

Ile Tyr Ala Asn Ser Asn Arg Pro Ser Gly Val Pro Asp Arg Phe Ser
    50                  55                  60

Gly Ser Lys Ser Gly Thr Ser Ala Ser Leu Ala Ile Ser Gly Leu Arg
65                  70                  75                  80

Ser Glu Asp Glu Ala Asp Tyr Tyr Cys Gly Ala Trp Asp Tyr Ser Leu
                85                  90                  95

Ser Ala Tyr Val Phe Gly Gly Gly Thr Lys Leu Thr Val Leu Arg Thr
            100                 105                 110

Val Ala Ala Pro Thr Val Ser Ile Phe Pro Pro Ser Ser Glu Gln Leu
        115                 120                 125

Thr Ser Gly Gly Ala Ser Val Val Cys Phe Leu Asn Asn Phe Tyr Pro
    130                 135                 140

Lys Asp Ile Asn Val Lys Trp Lys Ile Asp Gly Ser Glu Arg Gln Asn
145                 150                 155                 160

Gly Val Leu Asn Ser Trp Thr Asp Gln Asp Ser Lys Asp Ser Thr Tyr
                165                 170                 175

Ser Met Ser Ser Thr Leu Thr Leu Thr Lys Asp Glu Tyr Glu Arg His
            180                 185                 190

Asn Ser Tyr Thr Cys Glu Ala Thr His Lys Thr Ser Thr Ser Pro Ile
        195                 200                 205

Val Lys Ser Phe Asn Arg Asn Glu Cys
    210                 215

<210>    50
<211>    457

<212>    PRT
<213>    Artificial Sequence

<220>
<223>    GTC110-03 heavy chain_mouse IgG2 Fc_full sequence


<400>    50
Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Asn Tyr
            20                  25                  30

Ala Met Ser Trp Val Arg Gln Ala Pro Gly Lys Gly Leu Glu Trp Val
        35                  40                  45

Ser Ala Ile Tyr Pro Gly Gly Gly Ser Ile Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Asp Ile Leu Pro Cys Pro Trp Gly Arg Cys Tyr Tyr Asp Tyr
            100                 105                 110

Ala Met Asp Val Trp Gly Gln Gly Thr Leu Val Thr Val Ser Ser Ala
        115                 120                 125

Ser Thr Thr Ala Pro Ser Val Tyr Pro Leu Ala Pro Val Cys Gly Asp
    130                 135                 140

Thr Thr Gly Ser Ser Val Thr Leu Gly Cys Leu Val Lys Gly Tyr Phe
145                 150                 155                 160

Pro Glu Pro Val Thr Leu Thr Trp Asn Ser Gly Ser Leu Ser Ser Gly
                165                 170                 175

Val His Thr Phe Pro Ala Val Leu Gln Ser Asp Leu Tyr Thr Leu Ser
            180                 185                 190

Ser Ser Val Thr Val Thr Ser Ser Thr Trp Pro Ser Gln Ser Ile Thr
        195                 200                 205

Cys Asn Val Ala His Pro Ala Ser Ser Thr Lys Val Asp Lys Lys Ile
    210                 215                 220

Glu Pro Arg Gly Pro Thr Ile Lys Pro Cys Pro Pro Cys Lys Cys Pro
225                 230                 235                 240

Ala Pro Asn Leu Leu Gly Gly Pro Ser Val Phe Ile Phe Pro Pro Lys
                245                 250                 255

Ile Lys Asp Val Leu Met Ile Ser Leu Ser Pro Ile Val Thr Cys Val
            260                 265                 270

Val Val Asp Val Ser Glu Asp Asp Pro Asp Val Gln Ile Ser Trp Phe
        275                 280                 285

Val Asn Asn Val Glu Val His Thr Ala Gln Thr Gln Thr His Arg Glu

290                 295                 300

Asp Tyr Asn Ser Thr Leu Arg Val Val Ser Ala Leu Pro Ile Gln His
305                 310                 315                 320

Gln Asp Trp Met Ser Gly Lys Glu Phe Lys Cys Lys Val Asn Asn Lys
                325                 330                 335

Asp Leu Pro Ala Pro Ile Glu Arg Thr Ile Ser Lys Pro Lys Gly Ser
            340                 345                 350

Val Arg Ala Pro Gln Val Tyr Val Leu Pro Pro Pro Glu Glu Glu Met
        355                 360                 365

Thr Lys Lys Gln Val Thr Leu Thr Cys Met Val Thr Asp Phe Met Pro
    370                 375                 380

Glu Asp Ile Tyr Val Glu Trp Thr Asn Asn Gly Lys Thr Glu Leu Asn
385                 390                 395                 400

Tyr Lys Asn Thr Glu Pro Val Leu Asp Ser Asp Gly Ser Tyr Phe Met
                405                 410                 415

Tyr Ser Lys Leu Arg Val Glu Lys Lys Asn Trp Val Glu Arg Asn Ser
            420                 425                 430

Tyr Ser Cys Ser Val Val His Glu Gly Leu His Asn His His Thr Thr
        435                 440                 445

Lys Ser Phe Ser Arg Thr Pro Gly Lys
    450                 455


<210>    51
<211>    217
<212>    PRT
<213>    Artificial Sequence

<220>
<223>    GTC110-03 light chain_mouse IgG2 Fc_full sequence


<400>    51
Gln Ser Val Leu Thr Gln Pro Pro Ser Ala Ser Gly Thr Pro Gly Gln
1               5                   10                  15

Arg Val Thr Ile Ser Cys Ser Asp Ser Ser Ser Asn Ile Gly Ser Asn
            20                  25                  30

Thr Val Ser Trp Tyr Gln Gln Leu Pro Gly Thr Ala Pro Lys Leu Leu
        35                  40                  45

Ile Tyr Ala Asp Asn Asn Arg Pro Ser Gly Val Pro Asp Arg Phe Ser
    50                  55                  60

Gly Ser Lys Ser Gly Thr Ser Ala Ser Leu Ala Ile Ser Gly Leu Arg
65                  70                  75                  80

Ser Glu Asp Glu Ala Asp Tyr Tyr Cys Gly Thr Trp Asp Tyr Ser Leu
                85                  90                  95

Ser Gly Tyr Val Phe Gly Gly Gly Thr Lys Leu Thr Val Leu Arg Thr
            100                 105                 110

Val Ala Ala Pro Thr Val Ser Ile Phe Pro Pro Ser Ser Glu Gln Leu
        115                 120                 125

Thr Ser Gly Gly Ala Ser Val Val Cys Phe Leu Asn Asn Phe Tyr Pro
    130                 135                 140

Lys Asp Ile Asn Val Lys Trp Lys Ile Asp Gly Ser Glu Arg Gln Asn
145                 150                 155                 160

Gly Val Leu Asn Ser Trp Thr Asp Gln Asp Ser Lys Asp Ser Thr Tyr
                165                 170                 175

Ser Met Ser Ser Thr Leu Thr Leu Thr Lys Asp Glu Tyr Glu Arg His
            180                 185                 190

Asn Ser Tyr Thr Cys Glu Ala Thr His Lys Thr Ser Thr Ser Pro Ile
        195                 200                 205

Val Lys Ser Phe Asn Arg Asn Glu Cys
    210                 215


<210>    52
<211>    457
<212>    PRT
<213>    Artificial Sequence

<220>
<223>    GTC110-04 heavy chain_mouse IgG2 Fc_full sequence


<400>    52
Glu Val Gln Leu Leu Glu Ser Gly Gly Gly Leu Val Gln Pro Gly Gly
 1               5                  10                  15

Ser Leu Arg Leu Ser Cys Ala Ala Ser Gly Phe Thr Phe Ser Asn Tyr
            20                  25                  30

Ala Met Ser Trp Val Arg Gln Ala Pro Gly Lys Gly Leu Glu Trp Val
        35                  40                  45

Ser Val Ile Ser His Gly Gly Gly Ser Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Val Ile Ser Asn Cys His Leu Gly Val Cys Tyr Tyr Ser Asn
            100                 105                 110

Gly Met Asp Val Trp Gly Gln Gly Thr Leu Val Thr Val Ser Ser Ala
        115                 120                 125

Ser Thr Thr Ala Pro Ser Val Tyr Pro Leu Ala Pro Val Cys Gly Asp
    130                 135                 140

Thr Thr Gly Ser Ser Val Thr Leu Gly Cys Leu Val Lys Gly Tyr Phe
145                 150                 155                 160

Pro Glu Pro Val Thr Leu Thr Trp Asn Ser Gly Ser Leu Ser Ser Gly
                165                 170                 175

Val His Thr Phe Pro Ala Val Leu Gln Ser Asp Leu Tyr Thr Leu Ser
            180                 185                 190

Ser Ser Val Thr Val Thr Ser Ser Thr Trp Pro Ser Gln Ser Ile Thr
        195                 200                 205

Cys Asn Val Ala His Pro Ala Ser Ser Thr Lys Val Asp Lys Lys Ile
    210                 215                 220

Glu Pro Arg Gly Pro Thr Ile Lys Pro Cys Pro Pro Cys Lys Cys Pro
225                 230                 235                 240

Ala Pro Asn Leu Leu Gly Gly Pro Ser Val Phe Ile Phe Pro Pro Lys
                245                 250                 255

Ile Lys Asp Val Leu Met Ile Ser Leu Ser Pro Ile Val Thr Cys Val
            260                 265                 270

Val Val Asp Val Ser Glu Asp Asp Pro Asp Val Gln Ile Ser Trp Phe
        275                 280                 285

Val Asn Asn Val Glu Val His Thr Ala Gln Thr Gln Thr His Arg Glu
    290                 295                 300

Asp Tyr Asn Ser Thr Leu Arg Val Val Ser Ala Leu Pro Ile Gln His
305                 310                 315                 320

Gln Asp Trp Met Ser Gly Lys Glu Phe Lys Cys Lys Val Asn Asn Lys
                325                 330                 335

Asp Leu Pro Ala Pro Ile Glu Arg Thr Ile Ser Lys Pro Lys Gly Ser
            340                 345                 350

Val Arg Ala Pro Gln Val Tyr Val Leu Pro Pro Pro Glu Glu Glu Met
        355                 360                 365

Thr Lys Lys Gln Val Thr Leu Thr Cys Met Val Thr Asp Phe Met Pro
    370                 375                 380

Glu Asp Ile Tyr Val Glu Trp Thr Asn Asn Gly Lys Thr Glu Leu Asn
385                 390                 395                 400

Tyr Lys Asn Thr Glu Pro Val Leu Asp Ser Asp Gly Ser Tyr Phe Met
                405                 410                 415

Tyr Ser Lys Leu Arg Val Glu Lys Lys Asn Trp Val Glu Arg Asn Ser
            420                 425                 430

Tyr Ser Cys Ser Val Val His Glu Gly Leu His Asn His His Thr Thr
        435                 440                 445

Lys Ser Phe Ser Arg Thr Pro Gly Lys
    450                 455


<210>   53
<211>   217
<212>   PRT
<213>   Artificial Sequence

<220>
<223>    GTC110-04 light chain_mouse IgG2 Fc_full sequence

<400>    53
Gln Ser Val Leu Thr Gln Pro Pro Ser Ala Ser Gly Thr Pro Gly Gln
1               5                   10                  15

Arg Val Thr Ile Ser Cys Ser Gly Ser Ser Ser Asn Ile Gly Asn Asn
            20                  25                  30

Asp Val Tyr Trp Tyr Gln Gln Leu Pro Gly Thr Ala Pro Lys Leu Leu
        35                  40                  45

Ile Tyr Ser Asp Ser Gln Arg Pro Ser Gly Val Pro Asp Arg Phe Ser
    50                  55                  60

Gly Ser Lys Ser Gly Thr Ser Ala Ser Leu Ala Ile Ser Gly Leu Arg
65                  70                  75                  80

Ser Glu Asp Glu Ala Asp Tyr Tyr Cys Gly Thr Trp Asp Tyr Ser Leu
                85                  90                  95

Ser Gly Tyr Val Phe Gly Gly Gly Thr Lys Leu Thr Val Leu Arg Thr
            100                 105                 110

Val Ala Ala Pro Thr Val Ser Ile Phe Pro Pro Ser Ser Glu Gln Leu
        115                 120                 125

Thr Ser Gly Gly Ala Ser Val Val Cys Phe Leu Asn Asn Phe Tyr Pro
    130                 135                 140

Lys Asp Ile Asn Val Lys Trp Lys Ile Asp Gly Ser Glu Arg Gln Asn
145                 150                 155                 160

Gly Val Leu Asn Ser Trp Thr Asp Gln Asp Ser Lys Asp Ser Thr Tyr
                165                 170                 175

Ser Met Ser Ser Thr Leu Thr Leu Thr Lys Asp Glu Tyr Glu Arg His
            180                 185                 190

Asn Ser Tyr Thr Cys Glu Ala Thr His Lys Thr Ser Thr Ser Pro Ile
        195                 200                 205

Val Lys Ser Phe Asn Arg Asn Glu Cys
    210                 215

<210>    54
<211>    5
<212>    PRT
<213>    Artificial Sequence

<220>
<223>    Lrig-1 epitope

<400>    54
Asn Leu Ser Tyr Asn
1               5

<210>    55

<211>    6
<212>    PRT
<213>    Artificial Sequence

<220>
<223>    Lrig-1 epitope


<400>    55
Leu Asp Leu Asn Arg Asn
1               5


<210>    56
<211>    6
<212>    PRT
<213>    Artificial Sequence

<220>
<223>    Lrig-1 epitope


<400>    56
Leu Phe Leu Gln His Asn
1               5


<210>    57
<211>    6
<212>    PRT
<213>    Artificial Sequence

<220>
<223>    Lrig-1 epitope


<400>    57
Leu Asn Leu Ala Gly Asn
1               5


<210>    58
<211>    6
<212>    PRT
<213>    Artificial Sequence

<220>
<223>    Lrig-1 epitope


<400>    58
Leu Asp Leu Ser Leu Asn
1               5


<210>    59
<211>    6
<212>    PRT
<213>    Artificial Sequence

<220>
<223>    Lrig-1 epitope

<400>    59
Leu Arg Leu Ser Lys Asn
1               5

<210>    60
<211>    6
<212>    PRT
<213>    Artificial Sequence

<220>
<223>    Lrig-1 epitope

<400>    60
Leu Lys Leu Gln Arg Asn
1               5

<210>    61
<211>    6
<212>    PRT
<213>    Artificial Sequence

<220>
<223>    Lrig-1 epitope

<400>    61
Leu His Leu Glu Tyr Asn
1               5

<210>    62
<211>    6
<212>    PRT
<213>    Artificial Sequence

<220>
<223>    Lrig-1 epitope

<400>    62
Leu His Leu Ser Asn Asn
1               5

<210>    63
<211>    6
<212>    PRT
<213>    Artificial Sequence

<220>
<223>    Lrig-1 epitope

<400>    63
Leu Val Leu Ser Phe Asn
1               5

<210>    64

<211>    6
<212>    PRT
<213>    Artificial Sequence

<220>
<223>    Lrig-1 epitope


<400>    64
Leu Arg Leu Ser His Asn
1               5


<210>    65
<211>    6
<212>    PRT
<213>    Artificial Sequence

<220>
<223>    Lrig-1 epitope


<400>    65
Leu Asp Leu Asp His Asn
1               5


<210>    66
<211>    6
<212>    PRT
<213>    Artificial Sequence

<220>
<223>    Lrig-1 epitope


<400>    66
Leu Thr Leu Phe Gly Asn
1               5


<210>    67
<211>    6
<212>    PRT
<213>    Artificial Sequence

<220>
<223>    Lrig-1 epitope


<400>    67
Leu Asn Leu Gly Gly Asn
1               5


<210>    68
<211>    12
<212>    PRT
<213>    Artificial Sequence

<220>
<223>    Lrig-1 epitope

<400>    68
Trp Thr Arg Ser Leu Asn Leu Ser Tyr Asn Lys Leu
1               5                   10

<210>    69
<211>    14
<212>    PRT
<213>    Artificial Sequence

<220>
<223>    Lrig-1 epitope

<400>    69
Thr Glu Val Arg Asn Thr Cys Phe Pro His Gly Pro Pro Ile
1               5                   10

<210>    70
<211>    13
<212>    PRT
<213>    Artificial Sequence

<220>
<223>    Lrig-1 epitope

<400>    70
Arg Leu Thr Gln Leu Asp Leu Asn Arg Asn Arg Ile Arg
1               5                   10

<210>    71
<211>    15
<212>    PRT
<213>    Artificial Sequence

<220>
<223>    Lrig-1 epitope

<400>    71
Asp Leu Asn Arg Asn Arg Ile Arg Leu Ile Glu Gly Leu Thr Phe
1               5                   10                  15

<210>    72
<211>    11
<212>    PRT
<213>    Artificial Sequence

<220>
<223>    Lrig-1 epitope

<400>    72
Asn Ser Ile Ala Arg Ile His Arg Lys Gly Trp
1               5                   10

<210>    73

<211>     14
<212>     PRT
<213>     Artificial Sequence

<220>
<223>     Lrig-1 epitope


<400>     73
Trp Leu Pro Pro Trp Leu Ile Gly Arg Met Leu Gln Ala Phe
  1               5                   10


<210>     74
<211>     11
<212>     PRT
<213>     Artificial Sequence

<220>
<223>     Lrig-1 epitope


<400>     74
Arg Gln Val Thr Phe Gly His Glu Gly Arg Tyr
  1               5                   10


<210>     75
<211>     15
<212>     PRT
<213>     Artificial Sequence

<220>
<223>     Lrig-1 epitope


<400>     75
Phe Gly His Glu Gly Arg Tyr Gln Cys Val Ile Thr Asn His Phe
  1               5                   10                  15


<210>     76
<211>     15
<212>     PRT
<213>     Artificial Sequence

<220>
<223>     Lrig-1 epitope


<400>     76
Arg Leu Thr Val Asn Val Leu Pro Ser Phe Thr Lys Thr Pro His
  1               5                   10                  15


<210>     77
<211>     13
<212>     PRT
<213>     Artificial Sequence

<220>
<223>     Lrig-1 epitope

<400>    77
Arg Arg Met His Val Met Pro Asp Asp Asp Val Phe Phe
1               5                   10

<210>    78
<211>    15
<212>    PRT
<213>    Artificial Sequence

<220>
<223>    Lrig-1 epitope

<400>    78
Phe Phe Ile Thr Asp Val Lys Ile Asp Asp Ala Gly Val Tyr Ser
1               5                   10                  15

<210>    79
<211>    13
<212>    PRT
<213>    Artificial Sequence

<220>
<223>    Lrig-1 epitope

<400>    79
Lys Gly Asp Arg Pro Leu Ser Leu Thr Glu Arg His His
1               5                   10

<210>    80
<211>    24
<212>    DNA
<213>    Artificial Sequence

<220>
<223>    mouse Lrig-1 forward primer

<400>    80
gacggaattc agtgaggaga acct                                              24

<210>    81
<211>    24
<212>    DNA
<213>    Artificial Sequence

<220>
<223>    mouse Lrig-1 reverse primer

<400>    81
caactggtag tggcagcttg tagg                                              24

<210>    82
<211>    24
<212>    DNA

<213>    Artificial Sequence

<220>
<223>    mouse Lrig-2 forward primer


<400>    82
tcacaaggaa cattgtctga acca                                              24


<210>    83
<211>    24
<212>    DNA
<213>    Artificial Sequence

<220>
<223>    mouse Lrig-2 reverse primer


<400>    83
gcctgatcta acacatcctc ctca                                              24


<210>    84
<211>    24
<212>    DNA
<213>    Artificial Sequence

<220>
<223>    mouse Lrig-3 forward primer


<400>    84
cagcaccttg agctgaacag aaac                                              24


<210>    85
<211>    24
<212>    DNA
<213>    Artificial Sequence

<220>
<223>    mouse Lrig-3 reverse primer


<400>    85
ccagcctttg gtaatctcgg ttag                                              24


<210>    86
<211>    24
<212>    DNA
<213>    Artificial Sequence

<220>
<223>    mouse Foxp3 forward primer


<400>    86
ctttcaccta tcccaccctt atcc                                              24


<210>    87

<211>    24
<212>    DNA
<213>    Artificial Sequence

<220>
<223>    mouse Foxp3 reverse primer


<400>    87
attcatctac ggtccacact gctc                                              24


<210>    88
<211>    20
<212>    DNA
<213>    Artificial Sequence

<220>
<223>    ACTG1 Forward primer


<400>    88
ggcgtcatgg tgggcatggg                                                   20


<210>    89
<211>    20
<212>    DNA
<213>    Artificial Sequence

<220>
<223>    ACTG1 reverse primer


<400>    89
atggcgtggg gaagggcgta                                                   20


<210>    90
<211>    458
<212>    PRT
<213>    Artificial Sequence

<220>
<223>    GTC110-04 heavy chain_human IgG1


<400>    90
Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Asn Tyr
            20                  25                  30

Ala Met Ser Trp Val Arg Gln Ala Pro Gly Lys Gly Leu Glu Trp Val
        35                  40                  45

Ser Val Ile Ser His Gly Gly Gly Ser Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Val Ile Ser Asn Cys His Leu Gly Val Cys Tyr Tyr Ser Asn
            100                 105                 110

Gly Met Asp Val Trp Gly Gln Gly Thr Leu Val Thr Val Ser Ser Ala
        115                 120                 125

Ser Thr Lys Gly Pro Ser Val Phe Pro Leu Ala Pro Ser Ser Lys Ser
    130                 135                 140

Thr Ser Gly Gly Thr Ala Ala Leu Gly Cys Leu Val Lys Asp Tyr Phe
145                 150                 155                 160

Pro Glu Pro Val Thr Val Ser Trp Asn Ser Gly Ala Leu Thr Ser Gly
                165                 170                 175

Val His Thr Phe Pro Ala Val Leu Gln Ser Ser Gly Leu Tyr Ser Leu
            180                 185                 190

Ser Ser Val Val Thr Val Pro Ser Ser Ser Leu Gly Thr Gln Thr Tyr
        195                 200                 205

Ile Cys Asn Val Asn His Lys Pro Ser Asn Thr Lys Val Asp Lys Lys
    210                 215                 220

Val Glu Pro Lys Ser Cys Asp Lys Thr His Thr Cys Pro Pro Cys Pro
225                 230                 235                 240

Ala Pro Glu Leu Leu Gly Gly Pro Ser Val Phe Leu Phe Pro Pro Lys
                245                 250                 255

Pro Lys Asp Thr Leu Met Ile Ser Arg Thr Pro Glu Val Thr Cys Val
            260                 265                 270

Val Val Asp Val Ser His Glu Asp Pro Glu Val Lys Phe Asn Trp Tyr
        275                 280                 285

Val Asp Gly Val Glu Val His Asn Ala Lys Thr Lys Pro Arg Glu Glu
    290                 295                 300

Gln Tyr Asn Ser Thr Tyr Arg Val Val Ser Val Leu Thr Val Leu His
305                 310                 315                 320

Gln Asp Trp Leu Asn Gly Lys Glu Tyr Lys Cys Lys Val Ser Asn Lys
                325                 330                 335

Ala Leu Pro Ala Pro Ile Glu Lys Thr Ile Ser Lys Ala Lys Gly Gln
            340                 345                 350

Pro Arg Glu Pro Gln Val Tyr Thr Leu Pro Pro Ser Arg Glu Glu Met
        355                 360                 365

Thr Lys Asn Gln Val Ser Leu Thr Cys Leu Val Lys Gly Phe Tyr Pro
    370                 375                 380

Ser Asp Ile Ala Val Glu Trp Glu Ser Asn Gly Gln Pro Glu Asn Asn
385                 390                 395                 400

Tyr Lys Thr Thr Pro Pro Val Leu Asp Ser Asp Gly Ser Phe Phe Leu
                405                 410                 415

Tyr Ser Lys Leu Thr Val Asp Lys Ser Arg Trp Gln Gln Gly Asn Val
            420                 425                 430

Phe Ser Cys Ser Val Met His Glu Ala Leu His Asn His Tyr Thr Gln
        435                 440                 445

Lys Ser Leu Ser Leu Ser Pro Gly Lys Tyr
    450                 455

<210>    91
<211>    217
<212>    PRT
<213>    Artificial Sequence

<220>
<223>    GTC110-04 Light chain_human IgG1

<400>    91
Gln Ser Val Leu Thr Gln Pro Pro Ser Ala Ser Gly Thr Pro Gly Gln
  1               5                  10                  15

Arg Val Thr Ile Ser Cys Ser Gly Ser Ser Ser Asn Ile Gly Asn Asn
             20                  25                  30

Asp Val Tyr Trp Tyr Gln Gln Leu Pro Gly Thr Ala Pro Lys Leu Leu
         35                  40                  45

Ile Tyr Ser Asp Ser Gln Arg Pro Ser Gly Val Pro Asp Arg Phe Ser
     50                  55                  60

Gly Ser Lys Ser Gly Thr Ser Ala Ser Leu Ala Ile Ser Gly Leu Arg
 65                  70                  75                  80

Ser Glu Asp Glu Ala Asp Tyr Tyr Cys Gly Thr Trp Asp Tyr Ser Leu
                 85                  90                  95

Ser Gly Tyr Val Phe Gly Gly Gly Thr Lys Leu Thr Val Leu Arg Thr
            100                 105                 110

Val Ala Ala Pro Ser Val Phe Ile Phe Pro Pro Ser Asp Glu Gln Leu
        115                 120                 125

Lys Ser Gly Thr Ala Ser Val Val Cys Leu Leu Asn Asn Phe Tyr Pro
    130                 135                 140

Arg Glu Ala Lys Val Gln Trp Lys Val Asp Asn Ala Leu Gln Ser Gly
145                 150                 155                 160

Asn Ser Gln Glu Ser Val Thr Glu Gln Asp Ser Lys Asp Ser Thr Tyr
                165                 170                 175

Ser Leu Ser Ser Thr Leu Thr Leu Ser Lys Ala Asp Tyr Glu Lys His
            180                 185                 190

Lys Val Tyr Ala Cys Glu Val Thr His Gln Gly Leu Ser Ser Pro Val
        195                 200                 205

Thr Lys Ser Phe Asn Arg Gly Glu Cys
    210                 215

## Description

### [Technical Field]

The present invention relates to a composition for preventing, improving or treating immune checkpoint inhibitor-resistant cancer, including a binding molecule capable of specifically binding to a leucine-rich and immunoglobulin-like domains 1 (Lrig-1) protein, which is present on the surface of regulatory T cells (Treg cells), as an active ingredient.

### [Background Art]

Unlike conventional anticancer drugs that attack cancer itself, immunotherapy is a therapeutic agent that induces immune cells to selectively attack only cancer cells by injecting an artificial immune protein into the body to stimulate the immune system. Such immunotherapy includes an immune checkpoint inhibitor, immune cell therapy, and immunoviral therapy.

Antibody-based cancer therapy such as immunotherapy has potentially high specificity and high side effects, compared to conventional drugs. This is because the precise differentiation between normal and neoplastic cells by antibodies is possible, and their mode of action relies on a less toxic immunological anti-tumor mechanism such as entering cytotoxic immune cells and complementary activation.

A target for antibody-based therapy must have certain characteristics which become the basis for proper differentiation between normal cells and neoplastic cells. In development of effective and safe antibody therapy, it is needless to say that a target which is exclusively limited to tumor cells or not detected in normal tissue will be ideal. In another aspect, a high degree of overexpression can be the basis of a therapeutic window, and low side effects exemplified by human epidermal growth factor receptor type 2 (HER-2) as a result of gene amplification is an excellent target for an antibody, trastuzumab (Herceptin).

Other targets for antibodies which have been already approved for oncology therapy or under clinical development have unique characteristics of not being based on numerical overexpression of a target molecule on tumor cells. In the case of an antibody against the proteoglycan MUC-1, a peptide repeat epitope present in the backbone of a target is underglycosylated in tumor cells and changed to its normal counterpart. In the case of antibodies against CD20 (rituximab), CD52 (Kampat-IH) and CD22 (epratuzumab), antibody targets show comparable expression levels on tumor cells and normal lymphocytes. In this regard, since target-negative stem cells restore a normal lymphocyte repertoire, the removal of normal cells by antibodies may be tolerated. Another example of the different accessibility of antibody targets is carcinoembryonic antigen (CEA) and carboanhydrase IX (CA9). Both of these antigens are expressed in normal epithelia of the colon and kidney, respectively. However, since radiolabeled antibodies well distinguish tumor tissue and normal tissue, cytotoxic antibodies are well tolerated. This is probably because CA9 and CEA expression is limited only to the luminal side of normal epithelial tissue which an IgG antibody cannot access. An antigen epithelial cell adhesion molecule (Ep-CAM) also belongs to this category. As a homologous cell adhesion molecule to epithelial cells, it is located in the intercellular space. Interestingly, high affinity anti-Ep CAM antibodies are highly toxic, and antibodies with moderate affinity are well tolerated. This shows that not only the accessibility of an Ep-CAM target to normal cells, but also the kinetics of antibody binding may open a novel therapeutic window.

While eight antibodies have approved for treating neoplastic tissue-related diseases, most of them are directed against lymphoma and leukemia. Only three monoclonal antibodies, Herceptin, Avastin and Erbitux, work against solid tumors accounting for 90% or more of cancer mortality. mAbs having substantially existing medical demand and significant clinical advantages has already been provided, and their significant commercial success also motivated the development of a novel innovative approach having a good balance between antibody-based therapies for several groups of patients and their enhancement in efficacy.

However, in the case of lung cancer, the overall response rate (ORR) of PD-1/PD-L1 inhibitors is less than 15 to 20%. The phenomena that adequately explain the limitation of therapeutic efficacy using these PD-1/PD-L1 inhibitors have been suggested as immunosuppressive mechanisms created in a tumor microenvironment. Therefore, to improve the tumor microenvironment and increase the reaction rate of an immune checkpoint inhibitor, the number of T cells recognizing a tumor antigen increases, or a strategy using a TNFRS stimulator or a strategy of additionally injecting a specific antigen vaccine into a tumor has been used, but its efficacy is still insignificant.

### [Disclosure]

### [Technical Problem]

The present invention is directed to providing a composition for preventing, ameliorating or treating immune checkpoint inhibitor-resistant cancer, which includes a binding molecule specifically binding to a leucine-rich and immunoglobulin-like domains 1 (Lrig-1) protein as an active ingredient.

The present invention is also directed to providing a composition for preventing, ameliorating or treating immune checkpoint inhibitor-resistant cancer, which includes an antibody-drug conjugate (ADC) as an active ingredient.

The present invention is also directed to providing a method of preventing or treating immune checkpoint inhibitor-resistant cancer, which includes administering a binding molecule specifically binding to a Lrig-1 protein into a subject at a pharmaceutically effective amount.

The present invention is also directed to providing a method of preventing or treating immune checkpoint inhibitor-resistant cancer, which includes administering an ADC into a subject at a pharmaceutically effective amount.

However, technical problems to be solved in the present invention are not limited to the above-described problems, and other problems which are not described herein will be fully understood by those of ordinary skill in the art from the following descriptions.

### [Technical Solution]

### 1. Composition for preventing, ameliorating or treating immune checkpoint inhibitor-resistant cancer, which includes binding molecule specifically binding to Lrig-1 protein as active ingredient

According to one embodiment of the present invention, a composition for preventing, ameliorating or treating immune checkpoint inhibitor-resistant cancer, which includes a binding molecule specifically binding to a leucine-rich and immunoglobulin-like domains 1 (Lrig-1) protein as an active ingredient is provided.

The composition of the present invention may be used as a pharmaceutical composition or food composition.
In the present invention, the binding molecule may suppress the function of Treg cells, that is, Treg cells belonging to tumor infiltrating lymphocytes (TILs) or activated Treg cells, thereby very effectively blocking a phenomenon of suppressing the function of effector T cells by the Treg cells, and thus immune evasion cancer, that is, cancer having resistance to an immune checkpoint inhibitor may be effectively prevented, improved or treated.

In the present invention, Treg cells on which the Lrig-1 protein is present may be TILs or activated Treg cells.

The "activated regulatory T cells (Treg cells)" used herein refer to Treg cells which suppress the induction and diffusion of effector T cells or whose down-regulatory function is activated. Unlike Treg cells on which the Lrgi-1 protein is not present, in the case having the Lrig-1 protein present on a surface, the function of suppressing the induction and diffusion of effector T cells may be activated.

The "tumor infiltrating lymphocyte (TIL)" used herein means a lymphocyte infiltrating cancer tissue or a tumor microenvironment (TME) after moving from the bloodstream to the site of tumor tissue. While the cytotoxic immune response may be induced by the TIL, in the case of Treg cells having a Lrig-1 protein thereon, the function of suppressing the action of effector T cells is activated (activated Treg cells), so the cytotoxic immune response can be rather suppressed.

The "resistance to an immune checkpoint inhibitor" used herein means a non-responding group which has no response to a drug from the beginning due to an immune checkpoint inhibitor, for example, a PD-1 inhibitor pembrolizumab or nivolumab) or a PD-L1 inhibitor (atezolizumab), or having a phenomenon in which the response to a drug is reduced or disappears through a mechanism of recognizing an immune checkpoint inhibitor as a foreign substance and inducing an immune response that removes this substance due to the long-term treatment of the drug.

The "cancer" used herein refers to a physiological condition characterized by typically uncontrolled cell growth in mammals. The cancer to be prevented, improved or treated in the present invention may be a solid tumor consisting of a mass generated by abnormal cell growth in a solid organ, in which the solid tumor may be stomach cancer, liver cancer, glioblastoma, ovarian cancer, colorectal cancer, head and neck cancer, bladder cancer, renal cell carcinoma, breast cancer, metastatic cancer, prostate cancer, pancreatic cancer, melanoma or lung cancer depending on the area of a solid organ, and preferably, melanoma, but the present invention is not limited thereto.

The "Lrig-1 protein" used herein is a transmembrane protein, which consists of 1091 amino acids on the surface of Treg cells, and has leucine-rich repeats (LRR) and three immunoglobulin-like domains on the extracellular- or lumen-side, a transmembrane sequence and a cytoplasmic tail. The *LRIG* gene family includes *LRIG1, LRIG2* and *LRIG3* genes, and amino acids are highly conserved between them. The *LRIG1* gene is highly expressed in normal skin, and may be expressed in basal and follicle cells to regulate the proliferation of epithelial stem cells. Therefore, it plays an important role in maintaining the homeostasis of the epidermis, and when absent, may develop into psoriasis or skin cancer. It has been reported that if chromosome 3p14.3 where the *LRIG1* gene is located is cut, there is a possibility of developing into cancer cells, in fact, it was confirmed that, in the case of renal cell carcinoma and cutaneous squamous cell carcinoma, the expression of the *LRIG1* gene is highly reduced. However, it has recently been found that cancers expressing the Lrig-1 protein account for only 20 to 30% of all cancers. Meanwhile, for the purpose of the present invention, the Lrig-1 protein may be a protein present in a human or mouse, but the present invention is not limited thereto.

In the present invention, the Lrig-1 protein may be a human-derived polypeptide represented by SEQ ID NO: 1, or a mouse-derived polypeptide represented by SEQ ID NO: 3, but the present invention is not limited thereto.

In the present invention, the Lrig-1 protein represented by SEQ ID NO: 1 may be encoded by a polynucleotide represented by SEQ ID NO: 2, but the present invention is not limited thereto.

In the present invention, the Lrig-1 protein represented by SEQ ID NO: 3 may be encoded by a polynucleotide represented by SEQ ID NO: 4, but the present invention is not limited thereto.

The "binding molecule" used herein refers to a variable domain, for example, an intact immunoglobulin including a monoclonal antibody such as a chimeric, humanized or human-derived monoclonal antibody, or an immunoglobulin binding to an antigen, such as an immunoglobulin fragment competing with an intact immunoglobulin for bonding with monomeric HA or trimeric HA of influenza A virus. Regardless of a structure, an antigen-binding fragment binds to the same antigen recognized by an intact immunoglobulin. The antigen-binding fragment may be a peptide or polypeptide containing two or more consecutive groups, 20 or more consecutive amino acid residues, 25 or more consecutive amino acid residues, 30 or more consecutive amino acid residues, 35 or more consecutive amino acid residues, 40 or more consecutive amino acid residues, 50 or more consecutive amino acid residues, 60 or more consecutive amino acid residues, 70 or more consecutive amino acid residues, 80 or more consecutive amino acid residues, 90 or more consecutive amino acid residues, 100 or more consecutive amino acid residues, 125 or more consecutive amino acid residues, 150 or more consecutive amino acid residues, 175 or more consecutive amino acid residues, 200 or more consecutive amino acid residues, or 250 or more consecutive amino acid residues of the amino acid sequence of a binding molecule.

The "antigen-binding fragment" used herein includes particularly Fab, F(ab'), F(ab')2, Fv, dAb, Fd, a complementarity-determining region (CDR) fragment, a single-chain antibody (scFv), a bivalent single-chain antibody, a single-chain phage antibody, an unibody, a diabody, a triabody, a tetrabody, and a polypeptide containing one or more fragments of an immunoglobulin sufficient for binding to a specific antigen as a polypeptide. The fragment may be produced by enzymatic or chemical breakdown of a synthetic or intact immunoglobulin, or produced by a genetic engineering technique such as recombinant DNA technology. The production method is well known in the art.

In the present invention, the binding molecule may be an antibody or a fragment thereof.

In the present invention, the antibody may be a chimeric antibody, a humanized antibody, a human antibody, a bivalent antibody, a bispecific antibody, a minibody, a domain antibody, a bispecific antibody, an antibody mimetic, a unibody, a diabody, a triabody, a tetrabody or a fragment thereof, but the present invention is not limited thereto.

In the present invention, the "antibody" refers to a protein molecule serving as a receptor specifically recognizing an antibody, which includes an immunoglobulin molecule with immunological reactivity to a specific antigen. For the purpose of the present invention, the antigen may be a Lrig-1 protein present on the surface of Treg cells. Preferably, the antibody may be one that specifically recognizes a leucine-rich region or immunoglobulin-like domain of the Lrig-1 protein, but the present invention is not limited thereto.

In the present invention, the antibody includes not only a complete form having two full-length light chains and two full-length heavy chains, but also a functional fragment of an antibody molecule. The functional fragment of the antibody molecule may be a fragment retaining at least an antigen-binding function, and include Fab, F(ab'), F(ab')2 and Fv.

In the present invention, the antibody may be an antagonist antibody.

In the present invention, the "antagonist antibody" refers to an antibody which inhibits a biological action or activity in cells caused by binding between an antagonist antibody and a ligand by binding to a specific molecule on the surface of cells or in cells. For example, in the present invention, the antagonist antibody may bind to a Lrig-1 protein present on the surface of Treg cells, thereby reducing or inhibiting one or more biological actions of the Lrig-1 protein in cells or between cells.

In the present invention, the "immunoglobulin" has heavy chains and light chains, and each of the heavy chains and the light chains has a constant region and a variable region. A variable region of the light and heavy chains includes three hypervariable regions called complementarity determining regions (hereinafter, "CDRs") and four framework regions. The CDRs mainly serve to bind to an epitope of an antibody. The CDRs in each chain are typically referred to sequentially as CDR1, CDR2 and CDR3, starting from the N-terminus, and identified by the chain in which a particular CDR is located.

In addition, in the present invention, the "monoclonal antibody" is referred to as an antibody molecule having a single molecule composition obtained from substantially the same antibody population, and exhibits single binding specificity and affinity for a particular epitope.

In the present invention, the "full-length antibody" has a structure with two full-length light chains and two full-length heavy chains, and each light chain is connected with a heavy chain by a disulfide bond, and includes IgA, IgD, IgE, IgM, and IgG. The IgG includes IgG1, IgG2, IgG3 and IgG4 as subtypes.

In addition, in the present invention, the "functional fragment of an antibody" means a fragment retaining an antigen-binding function, and includes Fab, Fab', F(ab')₂ and Fv. The Fab is a structure including one variable region of each of the light and heavy chains, a constant region of the light chain and the first constant region (CH1 domain) of the heavy chain, and has one antigen-binding site. In addition, Fab' differs from Fab in that it has a hinge region containing one or more cysteine residues at the C-terminus of the CH1 domain of the heavy chain. The F(ab')₂ antibody is generated by forming a disulfide bond of a cysteine residue of the hinge region of Fab'. A variable fragment (Fv) means a minimal antibody fragment only having a heavy chain variable region and a light chain variable region. Double-stranded Fv (dsFv) has a heavy chain and a light chain, which are connected by a disulfide bond, and single-stranded Fv (scFv) has a variable region of the heavy chain and a variable region of a light chain, which are generally connected with a peptide linker by a covalent bond. The antibody fragment may obtain a Fab or F(ab')₂ fragment when a protease, for example, papain or pepsin is used, and may be produced by genetic recombination technology.

In the present invention, the "chimeric antibody" is an antibody made by the recombination of a variable region of a mouse antibody and a constant region of a human antibody, and is an antibody greatly improved in immune response compared to a mouse antibody.

In the present invention, the "humanized antibody" refers to an antibody in which the protein sequence of an antibody derived from a non-human species is modified to be similar to an antibody variant naturally produced in a human. For example, the humanized antibody may produce a humanized variable region by recombining a mouse-derived CDR with a human antibody-derived FR, and then a humanized antibody may be produced by recombining the resulting humanized variable region with a constant region of a preferable human antibody.

In the present invention, the "unibody" is produced by technology of producing a stable small antibody format to exhibit a therapeutic effect for a longer period of time than a general antibody, and the unibody may be produced to have only one region capable of binding to a target by removing a hinge region of an IgG4 antibody. Compared to the full-length IgG4 antibody, the unibody has very excellent stability. The unibody may be produced with reference to prior patents and literature.

In the present invention, the binding molecule may be a binding molecule which includes
a heavy chain variable region including a heavy chain CDR1 consisting of an amino acid sequence selected from the group consisting of SEQ ID NOs: 5, 13, 21 and 29; a heavy chain CDR2 consisting of an amino acid sequence selected from the group consisting of SEQ ID NOs: 6, 14, 22 and 30; and a heavy chain CDR3 consisting of an amino acid sequence selected from the group consisting of SEQ ID NO: 7, 15, 23 and 31; and
a light chain variable region including a light chain CDR1 consisting of an amino acid sequence selected from the group consisting of SEQ ID NO: 8, 16, 24 and 32; a light chain CDR2 consisting of an amino acid sequence selected from the group consisting of SEQ ID NO: 9, 17, 25 and 33; and a light chain CDR3 consisting of an amino acid sequence selected from the group consisting of SEQ ID NO: 10, 18, 26 and 34.

In the present invention, the binding molecule may be a binding molecule which includes a heavy chain variable region selected from the group consisting of
(a) a heavy chain variable region including a heavy chain CDR1 represented by SEQ ID NO: 5, a heavy chain CDR2 represented by SEQ ID NO: 6, and a heavy chain CDR3 represented by SEQ ID NO: 7;
(b) a heavy chain variable region including a heavy chain CDR1 represented by SEQ ID NO: 13, a heavy chain CDR2 represented by SEQ ID NO: 14, and a heavy chain CDR3 represented by SEQ ID NO: 15;
(c) a heavy chain variable region including a heavy chain CDR1 represented by SEQ ID NO: 21, a heavy chain CDR2 represented by SEQ ID NO: 22, and a heavy chain CDR3 represented by SEQ ID NO: 23; and
(d) a heavy chain variable region including a heavy chain CDR1 represented by SEQ ID NO: 29, a heavy chain CDR2 represented by SEQ ID NO: 30, and a heavy chain CDR3 represented by SEQ ID NO: 31; and
   a light chain variable region selected from the group consisting of (e) a light chain variable region including a light chain CDR1 represented by SEQ ID NO: 8, a light chain CDR2 represented by SEQ ID NO: 9, and a light chain CDR3 represented by SEQ ID NO: 10;
(f) a light chain variable region including a light chain CDR1 represented by SEQ ID NO: 16, a light chain CDR2 represented by SEQ ID NO: 17, and a light chain CDR3 represented by SEQ ID NO: 18;
(g) a light chain variable region including a light chain CDR1 represented by SEQ ID NO: 24, a light chain CDR2 represented by SEQ ID NO: 25, and a light chain CDR3 represented by SEQ ID NO: 26; and
(h) a light chain variable region including a light chain CDR1 represented by SEQ ID NO: 32, a light chain CDR2 represented by SEQ ID NO: 33, and a light chain CDR3 represented by SEQ ID NO: 34.

In the present invention, the binding molecule may be a binding molecule selected from the group consisting of
(1) a binding molecule which includes a heavy chain variable region including a heavy chain CDR1 represented by SEQ ID NO: 5, a heavy chain CDR2 represented by SEQ ID NO: 6, and a heavy chain CDR3 represented by SEQ ID NO: 7; and a light chain variable region including a light chain CDR1 represented by SEQ ID NO: 8, a light chain CDR2 represented by SEQ ID NO: 9, and a light chain CDR3 represented by SEQ ID NO: 10;
(2) a binding molecule which includes a heavy chain variable region including a heavy chain CDR1 represented by SEQ ID NO: 13, a heavy chain CDR2 represented by SEQ ID NO: 14, and a heavy chain CDR3 represented by SEQ ID NO: 15; and a light chain variable region including a light chain CDR1 represented by SEQ ID NO: 16, a light chain CDR2 represented by SEQ ID NO: 17, and a light chain CDR3 represented by SEQ ID NO: 18;
(3) a binding molecule which includes a heavy chain variable region including a heavy chain CDR1 represented by SEQ ID NO: 21, a heavy chain CDR2 represented by SEQ ID NO: 22, and a heavy chain CDR3 represented by SEQ ID NO: 23; and a light chain variable region including a light chain CDR1 represented by SEQ ID NO: 24, a light chain CDR2 represented by SEQ ID NO: 25, and a light chain CDR3 represented by SEQ ID NO: 26; and
(4) a binding molecule which includes a heavy chain variable region including a heavy chain CDR1 represented by SEQ ID NO: 29, a heavy chain CDR2 represented by SEQ ID NO: 30, and a heavy chain CDR3 represented by SEQ ID NO: 31; and a light chain variable region including a light chain CDR1 represented by SEQ ID NO: 32, a light chain CDR2 represented by SEQ ID NO: 33, and a light chain CDR3 represented by SEQ ID NO: 34.

In the present invention, the binding molecule may be a binding molecule which includes
a heavy chain variable region consisting of any one amino acid sequence selected from the group consisting of SEQ ID NOs: 11, 19, 27 and 35; and
a light chain variable region consisting of any one amino acid sequence selected from the group consisting of SEQ ID NO: 12, 20, 28 and 36.

In the present invention, the binding molecule may be a binding molecule selected from the group consisting of
(1) a binding molecule including a heavy chain variable region represented by SEQ ID NO: 11, and a light chain variable region represented by SEQ ID NO: 12;
(2) a binding molecule including a heavy chain variable region represented by SEQ ID NO: 19, and a light chain variable region represented by SEQ ID NO: 20;
(3) a binding molecule including a heavy chain variable region represented by SEQ ID NO: 27, and a light chain variable region represented by SEQ ID NO: 28; and
(4) a binding molecule including a heavy chain variable region represented by SEQ ID NO: 35, and a light chain variable region represented by SEQ ID NO: 36.

In the present invention, the binding molecule may further include a fragment crystallization (Fc) region or a constant region. Here, the Fc region may be an Fc region of IgA, IgD, IgE, IgM, IgG1, IgG2, IgG3 or IgG4 antibody, or may be derived therefrom, or a hybrid Fc region.

In the present invention, the Fc region may be an Fc region of mammal-derived IgA, IgD, IgE, IgM, IgG1, IgG2, IgG3 or IgG4 antibody, and preferably, an Fc region of human-derived IgA, IgD, IgE, IgM, IgG1, IgG2, IgG3 or IgG4 antibody, but the present invention is not limited thereto.

As an example of the present invention, the constant region may be a mouse-derived IgG2a constant region represented by SEQ ID NO: 37, but the present invention is not limited thereto.

As an example of the present invention, the constant region may be a mouse-derived immunoglobulin kappa constant region represented by SEQ ID NO: 38, but the present invention is not limited thereto.

As an example of the present invention, the constant region may be a human-derived IgG1 constant region represented by SEQ ID NO: 39 or 40, but the present invention is not limited thereto.

As an example of the present invention, the constant region may be a human-derived immunoglobulin kappa constant region represented by SEQ ID NO: 41, but the present invention is not limited thereto.

As an example of the present invention, the constant region may be a human-derived IgG2 constant region represented by SEQ ID NO: 42, but the present invention is not limited thereto.

As an example of the present invention, the constant region may be a human-derived IgG3 constant region represented by SEQ ID NO: 43, but the present invention is not limited thereto.

As an example of the present invention, the constant region may be a human-derived IgG4 constant region represented by SEQ ID NO: 44, but the present invention is not limited thereto.

As an example of the present invention, the constant region may be a human-derived immunoglobulin lambda (λ) constant region, but the present invention is not limited thereto.

In the present invention, the "hybrid Fc" may be induced from the combination of human IgG subclasses or the combination of human IgD and IgG. The hybrid Fc may not only increase the serum half-life of the biologically active molecule when binding to a biologically active molecule or polypeptide, but also increase a polypeptide expression level when a nucleotide encoding a Fc-polypeptide-fused protein is expressed.

As an example of the present invention, the hybrid Fc region may be a hybrid Fc represented by SEQ ID NO: 45, but the present invention is not limited thereto.

In the binding molecule of the present invention, the Fc or constant region may be connected to the variable region by a linker. Here, a linker is connected to the C-terminus of the Fc region, and the N-terminus of the binding molecule of the present invention may be connected to the linker, but the present invention is not limited thereto.

In the present invention, the "linker" may include a sequence that can be cleaved by an enzyme overexpressed in tissue or cells of a target disease. In the case of cleavage by the overexpressed enzyme, a degradation in polypeptide activity by the Fc part may be effectively prevented. In the present invention, the linker is preferably a peptide linker consisting of 33 amino acids located at positions 282 to 314, and more preferably a peptide linker consisting of 13 amino acids located at positions 292 to 304 of human albumin most abundantly present in blood, and this part has a minimal possibility of inducing an immune response in the body, which is mostly exposed to the outside due to a three-dimensional structure. However, the present invention is not limited thereto.

The binding molecule of the present invention may further include a heavy chain constant region consisting of an amino acid sequence selected from the group consisting of SEQ ID NOs: 37, 39, 40, 42, 43, 44 and 45.

The binding molecule of the present invention may further include a light chain constant region consisting of an amino acid sequence represented by SEQ ID NO: 38 or 41.

The binding molecule of the present invention may further include
a heavy chain constant region consisting of an amino acid sequence represented by SEQ ID NO: 37; and
a light chain constant region consisting of an amino acid sequence represented by SEQ ID NO: 38.

The binding molecule of the present invention may further include
a heavy chain constant region consisting of an amino acid sequence represented by SEQ ID NO: 39, 40, 42, 43 or 44; and
a light chain constant region consisting of an amino acid sequence represented by SEQ ID NO: 41.

The binding molecule of the present invention may further include
a heavy chain constant region consisting of an amino acid sequence represented by SEQ ID NO: 45.

The binding molecule of the present invention may be
a binding molecule including a heavy chain represented by SEQ ID NO: 46 and a light chain represented by SEQ ID NO: 47;
a binding molecule including a heavy chain represented by SEQ ID NO: 48 and a light chain represented by SEQ ID NO: 49;
a binding molecule including a heavy chain represented by SEQ ID NO: 50 and a light chain represented by SEQ ID NO: 51; and
a binding molecule including a heavy chain represented by SEQ ID NO: 52 and a light chain represented by SEQ ID NO: 53.

The binding molecule of the present invention may be
a binding molecule including a heavy chain represented by SEQ ID NO: 90 and a light chain represented by SEQ ID NO: 91.

The binding molecule of the present invention is an antibody, but the present invention is not limited thereto. The antibody is a monoclonal antibody, a full-length antibody, or any antibody fragment having the ability to bind to a Lrig-1 protein and binding to a Lrig-1 antigenic determinant competing with the binding molecule of the present invention as a part of an antibody.

In the present invention, the binding molecule may bind to a Lrig-1 protein, and may be provided as a bispecific antibody or bispecific antigen-binding fragment which can bind to a different protein, other than the Lrig-1 protein.

In the present invention, the bispecific antibody and the bispecific antigen-binding fragment may include a binding molecule according to the present invention. As an example of the present invention, the bispecific antibody and the bispecific antigen-binding fragment may include an antigen-binding domain capable of binding to the Lrig-1 protein. Here, the antigen-binding domain capable of binding to the Lrig-1 may include a binding molecule according to the present invention or consist of the binding molecule.

The bispecific antibody and the bispecific antigen-binding fragment according to the present invention includes an antigen-binding domain, which is a binding molecule capable of binding to the Lrig-1 protein according to the present invention, and an antigen-binding domain capable of binding to a different target protein. Here, the antigen-binding domain capable of binding to a different target protein may be a protein different from the Lrig-1 protein, for example, PD-1 or a cell surface receptor, but the present invention is not limited thereto.

The bispecific antibody and the bispecific antigen-binding fragment according to the present invention may be provided in a format described as any suitable format. For example, the bispecific antibody or bispecific antigen-binding fragment may be a bispecific antibody conjugate (e.g., IgG2, F(ab')2 or CovX-body), a bispecific IgG or IgG-type molecule (e.g., IgG, scFv4-Ig, IgG-scFv, scFv-IgG, DVD-Ig, IgG-sVD, sVD-IgG, or 2 in 1-IgG, mAb2, or Tandemab common LC), an asymmetric bispecific IgG or IgG-type molecule (e.g., kih IgG, kih IgG common LC, CrossMab, kih IgG-scFab, mAb-Fv, a pair of charges or SEED-body), a small bispecific antibody molecule (e.g., diabody (Db), dsDb, DART, scDb, tandAbs, tandem scFv (taFv), tandem dAb/VHH, a triple body, a triple head, Fab-scFv, or F(ab')2-scFv2), a bispecific Fc and CH3-fusion protein (e.g., taFv-Fc, di-diabody, scDb-CH3, scFv-Fc-scFv, HCAb-VHH, scFv-kih-Fc, or scFv-kih-CH3), or a bispecific fusion protein (e.g., scFv2-albumin, scDb-albumin, taFv-toxin, DNL-Fab3, DNL-Fab4-IgG, DNL-Fab4-IgG-cytokine 2). Those of ordinary skill in the art may design and produce the bispecific antibody and the bispecific antigen-binding fragment according to the present invention by a known method.

In the present invention, a method of producing the bispecific antibody may include a reducing disulfide or non-reducing thioether bond and chemical crosslinking of an antibody or antibody fragment. For example, to produce a disulfide-bonded bispecific F(ab)₂ heterodimer, N-succinimidyl-3-(2-pyridyldithio)-propionate (SPDP) may be used to chemically crosslink a Fab fragment by, for example, an SH- group of a hinge region.

In addition, in the present invention, another method for producing the bispecific antibody includes a process of fusing an antibody-producing hybridoma with, for example, polyethylene glycol to produce quadroma cells capable of secreting a bispecific antibody.

In the present invention, the bispecific antibody and the bispecific antigen-binding fragment may be produced through recombination, for example, by the expression of a nucleic acid construct encoding a polypeptide for an antigen-binding molecule. For example, light chain and heavy chain variable domains for two antigen-binding regions (that is, light chain and heavy chain variable regions for an antigen-binding domain capable of binding to PD-1, and light chain and heavy chain variable domains for an antigen-binding domain capable of binding to a different target protein) were encoded, and a DNA construct including a sequence encoding an appropriate linker or dimerization domain between the antigen-binding domains may be manufactured by molecular cloning technology. A recombinant bispecific antibody may be then produced by the expression of the construct in appropriate host cells (e.g., mammalian host cells), and the subsequently expressed recombinant bispecific antibody may optionally be purified.

In the present invention, the antibody may be produced by an affinity maturation process in which a modified antibody with improved affinity for an antigen, compared to an unmodified parent antibody, is produced. The affinity-matured antibody may be produced by procedures known in the art.

The binding molecule according to the present invention may include a variant of the amino acid sequence as long as it specifically binds to the Lrig-1 protein. For example, in order to improve the binding affinity and/or other biological characteristics of an antibody, the amino acid sequence of the antibody may be changed. Such alteration includes, for example, the deletion, insertion or substitution at a reside on the amino acid sequence of an antibody.

In the present invention, amino acid alteration may be made based on the relative similarity of amino acid side chain substituents, for example, hydrophobicity, hydrophilicity, charge or size. Through the analyses for the size, shape and type of an amino acid side chain substituent, it can be seen that all of arginine, lysine and histidine are positively charged residues; alanine, glycine and serine have similar sizes; and phenylalanine, tryptophan and tyrosine have similar shapes. Accordingly, based on the above considerations, arginine, lysine and histidine; alanine, glycine and serine; and phenylalanine, tryptophan and tyrosine may be biologically functional equivalents.

In the present invention, to introduce the alteration, a hydropathic index of an amino acid may be considered. A hydropathic index may be assigned to each amino acid depending on hydrophobicity and a charge: isoleucine (+4.5); valine (+4.2); leucine (+3.8); phenylalanine (+2.8); cysteine (+2.5); methionine (+1.9); alanine (+1.8); glycine (-0.4); threonine (-0.7); serine (-0.8); tryptophan (-0.9); tyrosine (-1.3); proline (-1.6); histidine (-3.2); glutamate (-3.5); glutamine (-3.5); aspartate (-3.5); asparagine (-3.5); lysine (-3.9); and arginine (-4.5). To impart the interactive biological function of a protein, a hydropathic amino acid index is very important. It is a known fact that similar biological activity may be retained only by substitution with a similar hydropathic index. When the alteration is introduced with reference to the hydropathic indices, substitution is made between amino acids showing a hydropathic index difference within, preferably ±2, more preferably ± 1, and even more preferably ± 0.

It is also well known that substitution between amino acids having similar hydrophilicity values in the present invention results in proteins having equivalent biological activity. The following hydrophilicity value is assigned to each amino acid: arginine (+3.0); lysine (+3.0); aspartate (+3.0± 1); glutamate (+3.0±1); serine (+0.3); asparagine (+0.2); glutamine (+0.2); glycine (0); threonine (-0.4); proline (-0.5±1); alanine (-0.5); histidine (-0.5); cysteine (-1.0); methionine (-1.3); valine (-1.5); leucine (-1.8); isoleucine (-1.8); tyrosine (-2.3); phenylalanine (-2.5); and tryptophan (-3.4). When the alteration is introduced with reference to the hydrophilicity values, substitution is made between amino acids showing a difference in hydrophilicity value within preferably ±2, more preferably ± 1, and even more preferably ± 0.5.

In the present invention, amino acid replacement in a protein that does not entirely alter the activity of the molecule is known to the art, and the most commonly occurring replacements are the replacement between amino acids, such as Ala/Ser, Val/Ile, Asp/Glu, Thr/Ser, Ala/Gly, Ala/Thr, Ser/Asn, Ala/Val, Ser/Gly, Tyr/Phe, Ala/Pro, Lys/Arg, Asp/Asn, Leu/Ile, Leu/Val, and Gln/Glu.

In the present invention, a binding molecule specifically binding to the Lrig-1 protein may bind to an epitope including a polypeptide consisting of the amino acid sequence represented by the following Formula 1:

[Formula 1] Lx¹Lx²x³N

In Formula 1,
x¹ to x³ may each be independently a neutral amino acid, an acidic amino acid, a basic amino acid or an aromatic amino acid. Here, the neutral amino acid may be glycine (G), alanine (A), valine (V), leucine (L), isoleucine (I), serine (S) or threonine (T), the acidic amino acid may be aspartic acid (D), glutamic acid (E), asparagine (N) or glutamine (Q), the basic amino acid may be lysine (K), arginine (R) or histidine (H), and the aromatic amino acid may be phenylalanine (F) or tyrosine (Y).

As an example of the present invention, x¹ to x³ may each be independently an amino acid selected from the group consisting of asparagine (N), aspartic acid (D), serine (S), tyrosine (Y), arginine (R), phenylalanine (F), lysine (K), histidine (H), leucine (L), valine (V), threonine (T), alanine (A), glutamine (Q), glutamic acid (E) and glycine (G).

As another example of the present invention, x¹ may be an amino acid selected from the group consisting of asparagine (N), phenylalanine (F), aspartic acid (D), lysine (K), histidine (H), valine (V), arginine (R) and threonine (T), x² may be an amino acid selected from the group consisting of serine (S), glutamine (Q), alanine (A), asparagine (N), glutamic acid (E), aspartic acid (D), phenylalanine (F) and glycine (G), and x³ may be an amino acid selected from the group consisting of tyrosine (Y), histidine (H), glycine ( G), arginine (R), asparagine (N), leucine (L), lysine (K) and phenylalanine (F).

As still another example of the present invention, x¹ to x³ may each be independently an amino acid selected from the group consisting of asparagine (N), aspartic acid (D), serine (S), tyrosine (Y) and arginine (R).

As yet another example of the present invention, x¹ is asparagine (N) or aspartic acid (D), x² is serine (S) or asparagine (N), and x³ is tyrosine (Y) or arginine (R).

In the present invention, the epitope may include a polypeptide consisting of an amino acid sequence represented by Formula 1, which may be 10 to 20 mers, preferably 10 to 15 mers, and more preferably 11 to 14 mers.

In addition, in the present invention, in the epitope, the polypeptide consisting of the amino acid sequence represented by Formula 1 may be located at 3^{rd} to 6^{th} positions, preferably, 4^{th} to 6^{th} positions, and more preferably the 5^{th} position from the N-terminus of the epitope.

As an example of the present invention, the polypeptide consisting of the amino acid sequence represented by Formula 1 may be represented by any one amino acid sequence of SEQ ID NOs: 54 to 67 shown in Table 1 below, but the present invention is not limited thereto:

**[Table 1]**

| Sequence list | Sequence data |
|---|---|
| SEQ ID NO: 54 | LNLSYN |
| SEQ ID NO: 55 | LDLNRN |
| SEQ ID NO: 56 | LFLQHN |
| SEQ ID NO: 57 | LNLAGN |
| SEQ ID NO: 58 | LDLSLN |
| SEQ ID NO: 59 | LRLSKN |
| SEQ ID NO: 60 | LKLQRN |
| SEQ ID NO: 61 | LHLEYN |
| SEQ ID NO: 62 | LHLSNN |
| SEQ ID NO: 63 | LVLSFN |
| SEQ ID NO: 64 | LRLSHN |
| SEQ ID NO: 65 | LDLDHN |
| SEQ ID NO: 66 | LTLFGN |
| SEQ ID NO: 67 | LNLGGN |

In the present invention, an epitope of the Lrig-1 protein may include a polypeptide represented by any one amino acid sequence of SEQ ID NOs: 68 to 79 shown in Table 2 below:

**[Table 2]**

| Sequence list | Sequence data |
|---|---|
| SEQ ID NO: 68 | WTRSLNLSYNKL |
| SEQ ID NO: 69 | TEVRNTCFPHGPPI |
| SEQ ID NO: 70 | RLTQLDLNRNRIR |
| SEQ ID NO: 71 | DLNRNRIRLIEGLTF |
| SEQ ID NO: 72 | NSIARIHRKGW |
| SEQ ID NO: 73 | WLPPWLIGRMLQAF |
| SEQ ID NO: 74 | RQVTFGHEGRY |
| SEQ ID NO: 75 | FGHEGRYQCVITNHF |
| SEQ ID NO: 76 | RLTVNVLPSFTKTPH |
| SEQ ID NO: 77 | RRMHVMPDDDVFF |
| SEQ ID NO: 78 | FFITDVKIDDAGVYS |
| SEQ ID NO: 79 | KGDRPLSLTERHH |

In another example of the present invention, the epitope may be represented by an amino acid sequence of SEQ ID NO: 66 or 68, but the present invention is not limited thereto.

When considering alterations with the above-described biologically equivalent activity, it is construed that the binding molecule of the present invention includes a sequence exhibiting substantial identity to the sequence listed in the Sequence Listing.

The term "substantial identity" refers to a sequence having at least 61% homology, more preferably 70% homology, even more preferably 80% homology, and most preferably 90% homology when the sequence of the present invention and any different sequence are aligned in parallel as much as possible, and the aligned sequences are analyzed using an algorithm conventionally used in the art. An alignment method for sequence comparison is known in the art. Various methods and algorithms for alignment, for example, the NCBI Basic Local Alignment Search Tool (BLAST) can be accessed at the National Center for Biological Information (NBCI), and used in conjunction with sequencing programs such as blastp, blasm, blastx, tblastn and tblastx at BLSAT on the internet, http://www.ncbi.nlm.nih.gov/BLAST/. The sequence homology comparison method using this program may be checked online (http://www.ncbi.nlm.nih.gov/BLAST/ blast_help.html).

In the present invention, the binding molecule, preferably the antibody, may be produced by a conventional method for producing an antibody, but may be produced by affinity maturation.

In the present invention, the "affinity maturation" refers to the procedure in which activated B cells produce antibodies with increased affinity for an antigen in an immune response. For the purpose of the present invention, the affinity maturation may produce an antibody or antibody fragment produced by affinity maturation based on the principle of mutation and selection, like the process occurring in nature.

In the present invention, the anticancer agent may be used in combination with another known anticancer agent.

In the present invention, the "anticancer agent" may be selected from the group consisting of, for example, nitrogen mustard, imatinib, oxaliplatin, rituximab, erlotinib, neratinib, lapatinib, gefitinib, vandetanib, nirotinib, semasanib, bosutinib, axitinib, cediranib, restaurtinib, trastuzumab, gefitinib, bortezomib, sunitinib, carboplatin, sorafenib, bevacizumab, cisplatin, cetuximab, viscumalbum, asparaginase, tretinoin, hydroxycarbamide, dasatinib, estramstine, gemtuzumab ozogamicin, ibritumomab tusetan, heptaplastin, methylaminolevulinic acid, amsacrine, alemtuzumab, procarbazine, alprostadil, holmium nitrate chitosan, gemcitabine, doxyfluridine, pemetrexed, tegafur, capecitabine, gimeracin, oteracil, azacitidine, methotrexate, uracil, cytarabine, fluorouracil, fludarabine, enocitabine, flutamide, capecitabine, decitabine, mercaptopurine, thioguanine, cladribine, carmofer , raltitrexed, docetaxel, paclitaxel, irinotecan, belotecan, topotecan, vinorelbine, etoposide, vinblastine, idarubicin, mitomycin, bleromycin, dactinomycin, pyrarubicin, pepromycin, aclarubicin, temsirolimus, temozolomide, busulfan, ifosfamide, cyclophosphamide, melparan, altretmine, dacarbazine, thiotepa, nimustine, chlorambucil, mitolactol, leucovorin, tretonin, exemestane, aminoglutethimide, anagrelide, olaparib, nabelbine, fadrozole, tamoxifen, toremifene, testolactone, anastrozole, letrozole, vorozol, bicalutamide, lomustine, 5FU, vorinostat, entinostat, carmustine, a VEGF antibody (bevacizumab), an EGFR antibody (cetuximab), a HER-2 antibody (trastuzumab), a PD-1 inhibitor (pembrolizumab,r nivolumab), a PD-L1 inhibitor (atezolizumab, abelumab, durvalumab, STI-1014, CX-072), a CTLA-4 antibody (ipilimumab), a LAG-3 antibody (BMS-986016, IMP-731, IMP-321), a CD137 antibody (urelumab, PF-05082566), a GITR antibody(BMS-986153, BMS-986156, TRX-518, MK-4166), an IDO antagonist (INCB-024360, indoximod, NLG-919), an OXO antibody (MEDI-6383 or MEDI-6469), an OXO40L antagonist (RG-7888), a CD40 antagonist (lukatumumab, dacetuzumab) and a CD27 antibody (barlirumab), a CD20 antibody (rituximab), a CD52 antibody (Kampat-IH), a CD22 antibody (epratuzumab), CD25 antibody, a TIGIT antibody (tiragolumab), a CCR4 antibody, an FR4 antibody, CD15s antibody, a PI-16 antibody and a LAIR-1 antibody, but the present invention is not limited thereto.

In the present invention, the "prevention" or "improvement" refers to all actions involved in preventing, inhibiting or delaying symptoms of a disease using a pharmaceutical composition of the present invention without limitation.

In addition, in the present invention, the "treatment" may include all actions involved in alleviating or beneficially changing symptoms of a disease using the pharmaceutical composition of the present invention without limitation.

In the present invention, the pharmaceutical composition may be formulated in the form of a capsule, a tablet, a granule, an injection, an ointment, a powder or a drink, and the pharmaceutical composition may be aimed at humans.

The pharmaceutical composition of the present invention may be, but is not limited to, formulated in the form of an oral formulation such as a powder, granules, a capsule, a tablet or an aqueous suspension, a preparation for external use, a suppository and a sterile injectable solution according to a conventional method. The pharmaceutical composition of the present invention may include a pharmaceutically acceptable carrier. As pharmaceutically acceptable carriers, a binder, a lubricant, a disintegrant, an excipient, a solubilizer, a dispersant, a stabilizer, a suspending agent, a coloring agent and a flavor may be used for oral administration, a mixture of a buffer, a preservative, a pain relief agent, a solubilizer, an isotonic agent and a stabilizer may be used for an injectable, and a base, an excipient, a lubricant and a preservative may be used for local administration. The pharmaceutical composition of the present invention may be prepared in various forms by being mixed with the above-described pharmaceutically acceptable carrier. For example, for oral administration, the pharmaceutical composition of the present invention may be prepared in various dosage forms such as a tablet, a troche, a capsule, an elixir, a suspension, a syrup and a wafer, and for injectables, the pharmaceutical composition of the present invention may be prepared in a unit dose ampoule or multiple dose forms. In addition, the pharmaceutical composition of the present invention may be formulated as a solution, a suspension, a tablet, a capsule or a sustained-release preparation.

Meanwhile, examples of carriers, excipients and diluents suitable for preparation may include lactose, dextrose, sucrose, sorbitol, mannitol, xylitol, erythritol, maltitol, starch, acacia gum, alginate, gelatin, calcium phosphate, calcium silicate, cellulose, methyl cellulose, microcrystalline cellulose, polyvinylpyrrolidone, water, methyl hydroxybenzoate, propyl hydroxybenzoate, talc, magnesium stearate, and mineral oil. The examples of carriers, excipients and diluents may also include a filler, an anti-agglomerate, a glidant, a wetting agent, a fragrance, an emulsifier, and a preservative.

Administration routes for the pharmaceutical composition according to the present invention may include, but are not limited to, oral, intravenous, intramuscular, intraarterial, intramedullary, intrathecal, intracardiac, transdermal, subcutaneous, intraperitoneal, intranasal, enteral, local, sublingual or rectal administration. Oral or parenteral administration is preferable.

The term "parenteral" used herein means subcutaneous, intradermal, intravenous, intramuscular, intraarticular, intrasynovial, intrasternal, intrathecal, intralesional and intracranial injections or infusions. The pharmaceutical composition of the present invention may be administered in the form of a suppository for rectal administration.

The pharmaceutical composition of the present invention may vary according to various factors including the activity of a specific compound used, age, body weight, general health, sex, diet, administration time, administration route, excretion rate, drug formulation, and the severity of a specific disease to be prevented or treated, and a dosage of the pharmaceutical composition may be suitably selected by those of ordinary skill in the art depending on a patient's condition, body weight, the severity of a disease, a drug type, an administration route and an administration duration, and may be 0.0001 to 50 mg/kg or 0.001 to 50 mg/kg per day. The pharmaceutical composition of the present invention may be administered once a day or several times in divided portions. The dose does not limit the scope of the present invention in any way. The pharmaceutical composition according to the present invention may be formulated as a pill, a sugar-coated tablet, a capsule, a liquid, a gel, a syrup, a slurry or a suspension.

The food composition of the present invention may be prepared in the form of various types of foods, for example, a beverage, a gum, a tea, a vitamin complex, a powder, a granule, a tablet, a capsule, a snack, a rice cake, bread, etc.

In the present invention, when the active ingredient is contained in the food composition, it may be added in a proportion of 0.1 to 50% with respect to the total weight of the food composition, but the present invention is not limited thereto.

When the food composition of the present invention is prepared in the form of a beverage, there is no particular limitation other than including the food composition in the indicated proportion, and like a typical beverage, various sweeteners or natural carbohydrates may be contained as additive ingredients. Specifically, examples of the above-mentioned natural carbohydrates include conventional sugars, for example, monosaccharides such as glucose, fructose, etc.; disaccharides such as maltose, sucrose, etc.; and polysaccharides such as dextrin, cyclodextrin, etc., and sugar alcohols such as xylitol, sorbitol, erythritol, etc. As the sweeteners, natural sweeteners [thaumatin, stevia extract (e.g., rebaudioside A, glycyrrhizin, etc.)] and synthetic sweeteners (saccharin, aspartame, etc.) may be advantageously used.

The food composition of the present invention may further include a variety of nutrients, vitamins, minerals (electrolytes), flavoring agents including synthetic and natural flavoring agents, coloring agents and fillers (cheese, chocolate, etc.), pectic acid and a salt thereof, alginic acid and a salt thereof, an organic acid, protective colloid thickening agents, pH modifiers, stabilizers, preservatives, glycerin, alcohols, or carbonating agents used in carbonated beverages.

In the present invention, the above ingredients may be used alone or in combination. The proportions of the additives are not the key factor of the present invention, but may be selected within a range of 0.1 to about 50 parts by weight with respect to 100 parts by weight of the food composition of the present invention. However, the present invention is not limited thereto.

### 2. Composition for preventing, improving or treating immune checkpoint inhibitor-resistant cancer, including antibody-drug conjugate as active ingredient

According to another embodiment of the present invention, a composition including an antibody-drug conjugate including an antibody or antigen-binding fragment specifically binding to a Lrig-1 protein; and a drug, as an active ingredient, is provided.

According to the present invention, in a pharmaceutical composition for preventing, improving or treating immune checkpoint inhibitor-resistant cancer, including the antibody-drug conjugate as an active ingredient, the descriptions relating to antibodies, resistance to an immune checkpoint inhibitor, cancer, prevention, improvement, etc. are the same as described above, so the description will be omitted.

In the present invention, the "antibody-drug conjugate (ADC)" refers to a form in which a drug is chemically linked to an antibody without degradation in biological activity of the antibody and the drug. In the present invention, the antibody-drug conjugate is a form in which a drug is linked to an amino acid residue of the N-terminus of a heavy chain and/or a light chain of an antibody, and specifically, a form in which a drug is linked to an α-amine group at the N-terminus of a heavy chain and/or a light chain of an antibody.

In the present invention, the "drug" may refer to any material having specific biological activity in cells, and is a concept including DNA, RNA or a peptide. The drug may be a form including a reactive group that can be crosslinked by reaction with an α-amino group, or a form to which a linker including a reactive group capable of crosslinking by reaction with an α-amino group is connected.

In the present invention, the type of reactive group capable of crosslinking by reaction with an α-amine group is not particularly limited as long as it is able to crosslink by reaction with an α-amine group at the N-terminus of a heavy or light chain of an antibody, and includes all types reacting with an amine group, which are known in the art. As an example, the reactive group may be any one of an isothiocyanate, an isocyanate, an acyl azide, a NHS ester, a sulfonyl chloride, an aldehyde, a glyoxal, an epoxide, an oxirane, a carbonate, an aryl halide, an imidoester, a carbodiimide, an anhydride and a fluorophenyl ester, but the present invention is not limited thereto.

In the present invention, the anticancer agent may be, but is not limited to, selected from the group selected from nitrogen mustard, imatinib, oxaliplatin, rituximab, erlotinib, neratinib, lapatinib, gefitinib, vandetanib, nilotinib, semasanib, bosutinib, axitinib, cediranib, lestaurtinib, trastuzumab, gefitinib, bortezomib, sunitinib, carboplatin, sorafenib, bevacizumab, cisplatin, cetuximab, viscumalbum, asparaginase, tretinoin, hydroxycarbamide, dasatinib, estramustine, gemtuzumab ozogamicin, ibritumomab tucetan, heptaplastin, methylaminolevulinic acid, amsacrine, alemtuzumab, procarbazine, alprostadil, holmium nitrate chitosan, gemcitabine, doxyfluridine, pemetrexed, tegafur, capecitabine, gimeracin, oteracil, azacitidine, methotrexate, uracil, cytarabine, fluorouracil, fludarabine, enocitabine, flutamide, capecitabine, decitabine, mercaptopurine, thioguanine, cladribine, carmopher, raltitrexed, docetaxed, paclitaxel, irinotecan, belotecan, tepotecan, vinorelbine, etoposide, vinblastine, idarubicin, mitomycin, bleomycin, dactinomycin, pyrarubicin, aclarubicin, pepromycin, temsirolimus, temozolomide, busulfan, ifosfamide, cyclophosphamide, melpharan, altretmine, dacarbazine, thiotepa, nimustine, chlorambucil, mitolactol, leucovorin, tretonin, exemestane, aminoglutethimide, anagrelide, olaparib, nabelbine, fadrozole, tamoxifen, toremifene, testolactone, anastrozole, letrozole, vorozole, bicalutamide, lomustine, 5FU, vorinostat, entinostat and carmustine.

### 3. Method of preventing or treating immune checkpoint inhibitor-resistant cancer, including binding molecule specifically binding to Lrig-1 protein as active ingredient

According to still another embodiment of the present invention, a method of preventing or treating immune checkpoint inhibitor-resistant cancer, including administering a binding molecule specifically binding to a Lrig-1 protein to a subject at a pharmaceutically effective amount, is provided.

In the prevention or treatment method of the present invention, the descriptions relating to a Lrig-1 protein, a binding molecule, immune checkpoint inhibitor resistance, cancer, treatment and prevention are the same as described above in "*1*. *Composition for preventing, improving or treating immune checkpoint inhibitor-resistant cancer, including binding molecule as active ingredient,"* and thus will be omitted.

The "subject" used herein is an individual suspected of having a disease, which includes mammals including a human, a mouse and livestock that have or may develop the corresponding disease, but subjects that can be treated with the binding molecule according to the present invention are included without limitation.

The method of the present invention may include administering the binding molecule according to the present invention at a pharmaceutically effective amount. A suitable total daily dosage may be determined by a physician within the range of sound medical judgment, and administered once or divided into several doses. However, for the purpose of the present invention, a specific therapeutically effective amount for a specific patient is preferably applied depending on various factors including, the type and degree of response to be accomplished, a composition including the specific active ingredient, which can include another agent in some cases, a patient's age, body weight, a general health condition, gender and diet, administration time, an administration route, a secretion rate of the composition including the active ingredient, a treatment period and a drug used with or concurrently with a drug, and similar factors well known in the medical field.

The method of preventing or treating immune checkpoint inhibitor-resistant cancer according to the present invention may be a combination therapy, which further includes administering a compound or material with therapeutic activity for one or more diseases, but the present invention is not limited thereto.

It should be understood that the "combination" used herein means concurrent, separate or sequential administration. In the case of sequential or separate administration, the interval of the administration of a secondary component should be such that the beneficial effect of the combination therapy is not lost.

In the present invention, the dosage of the binding molecule may be about 0.0001 µg to 500 mg per kg of a patient's body weight, but the present invention is not limited thereto.

### 4. Method of preventing or treating immune checkpoint inhibitor-resistant cancer, including antibody-drug conjugate as active ingredient

According to yet another embodiment of the present invention, a method of preventing or treating immune checkpoint inhibitor-resistant cancer, which includes administering a pharmaceutically effective amount of an antibody-drug conjugate containing an antibody or antigen-binding fragment specifically binding to a Lrig-1 protein; and a drug to a subject, is provided.

In the prevention or treatment method of the present invention, the descriptions relating to a Lrig-1 protein, a binding molecule, immune checkpoint inhibitor resistance, cancer, treatment and prevention are the same as described above in "*1*. *Composition for preventing, improving or treating immune checkpoint inhibitor-resistant cancer, including binding molecule as active ingredient,"* the description relating to an antibody-drug conjugate is the same as described above in "2. *Composition for preventing, improving or treating immune checkpoint inhibitor-resistant cancer, including antibody-drug conjugate as active ingredient,*" the descriptions relating to a subject, combination therapy and treatment are the same as described above in *"3. Method of preventing or treating immune checkpoint inhibitor-resistant cancer, including binding molecule as active ingredient,"* and thus will be omitted.

### [Advantageous Effects]

A binding molecule, and preferably, an antibody, which is specific for a Lrig-1 protein, according to the present invention can effectively prevent, improve or treat immune evasion cancer, that is, cancer with resistance to an immune checkpoint inhibitor by very effectively blocking a phenomenon in which the function of effector T cells is inhibited by regulatory T cells (Treg cells) by inhibiting the function of Treg cells in which the Lrig-1 protein is expressed at a high level, that is, Treg cells belonging to tumor infiltrating lymphocytes (TILs) or activated Treg cells.

### [Description of Drawings]

FIG. 1 shows the structure of a Lrig-1 protein according to one embodiment of the present invention.
FIG. 2 shows the structure of a Lrig-1 protein according to one embodiment of the present invention.
FIG. 3 shows the expression level of a Lrig-1 protein according to one embodiment of the present invention.
FIG. 4 shows the expression level of Lrig-1 mRNA according to one embodiment of the present invention.
FIG. 5 shows the expression level of Lrig-1 mRNA according to one embodiment of the present invention.
FIG. 6 shows the expression levels of Lrig-1, Lrig-2 and Lrig-3 mRNAs according to one embodiment of the present invention.
FIG. 7 shows the comparison result of the expression levels of a Lrig-1 protein in regulatory T cells (Treg cells) and non-Treg cells according to one embodiment of the present invention.
FIGS. 8A and 8B show the expression of a Lrig-1 protein on the surface of Treg cells according to one embodiment of the present invention.
FIG. 9 shows the result of analyzing the binding strength of Lrig-1 protein-specific monoclonal antibodies (GTC110-01, GTC110-02, GTC110-03 and GTC110-04) to a Lrig-1 protein according to one embodiment of the present invention.
FIG. 10 shows the result of epitope mapping of 10 µg/ml of a monoclonal antibody (GTC110-04) against a Lrig-1 protein using a microarray according to one embodiment.
FIG. 11 shows the result of epitope mapping of 100 µg/ml of a monoclonal antibody (GTC110-04) against a Lrig-1 protein using a microarray according to one embodiment of the present invention.
FIG. 12 shows the result of epitope mapping of a monoclonal antibody (GTC110-04) against a Lrig-1 protein using a microarray according to one embodiment of the present invention. Here, human antibody 04 denotes the monoclonal antibody GTC110-04.
FIG. 13 shows the result of analyzing a Lrig-1 protein-induced Stat3 phosphorylation-regulating mechanism in Treg cells of Lrig-1 protein-specific monoclonal antibodies (GTC110-01, GTC110-02, GTC110-03 and GTC110-04) according to one embodiment of the present invention. Here, 01 denotes GTC110-01, 02 denotes GTC110-02, 03 denotes GTC110-03, and 04 denotes GTC110-04.
FIGS. 14A and 14B show the results of confirming the effector T cell inhibitory effect of Treg cells according to the expression level of a Lrig-1 protein according to one embodiment of the present invention.
FIG. 15 is a schematic diagram illustrating an experiment design for an immune checkpoint inhibitor-resistant cancer treatment effect using a Lrig-1 protein-specific monoclonal antibody (GTC110-04) or PD-1 antibody according to one embodiment of the present invention.
FIG. 16 shows the result of confirming a decrease in tumor size by the treatment of immune checkpoint inhibitor-resistant cancer using a Lrig-1 protein-specific monoclonal antibody (GTC110-04) or PD-1 antibody.
FIG. 17 shows the result of confirming the cell distribution of tumor infiltrating lymphocytes by the treatment of immune checkpoint inhibitor-resistant cancer using a Lrig-1 protein-specific monoclonal antibody (GTC110-04) or PD-1 antibody through fluorescence-activated cell sorting (FACS) according to one embodiment of the present invention.

### [Modes of the Invention]

One embodiment of the present invention relates to a pharmaceutical composition for preventing or treating immune checkpoint inhibitor-resistant cancer, which includes a binding molecule specifically binding to a leucine-rich and immunoglobulin-like domains 1 (Lrig-1) protein as an active ingredient.

Another embodiment of the present invention relates to a pharmaceutical composition for preventing or treating immune checkpoint inhibitor-resistant cancer, which includes an antibody-drug conjugate including an antibody or antigen-binding fragment, which specifically binds to a Lrig-1 protein; and a drug as an active ingredient.

Still another embodiment of the present invention relates to a method of preventing or treating immune checkpoint inhibitor-resistant cancer, which includes administering a binding molecule specifically binding to a Lrig-1 protein to a subject at a pharmaceutically effective amount.

Yet another embodiment of the present invention relates to a method of preventing or treating immune checkpoint inhibitor-resistant cancer, which includes administering an antibody-drug conjugate including an antibody or antigen-binding fragment specifically binding to a Lrig-1 protein; and a drug to a subject at a pharmaceutically effective amount.

Hereinafter, the present invention will be described in detail with reference to the following examples. The following examples are merely provided to exemplify the present invention, and the contents of the present invention are not limited to the following examples.

### Examples

### [Preparation Example 11 Culture of T cell subsets

To confirm whether a Lrig-1 protein is expressed only in regulatory T cells (Treg cells, also referred to as "Treg"), T cell subsets, for example, Th0, Th1, Th2, Th17 and iTreg cells, were prepared. Here, the iTreg cells refers to cells artificially inducing differentiation in a medium having the following composition, unlike naturally isolated nTreg cells.

The T cell subsets induced differentiation into individual cells through 72 hour-culture in 5% CO₂ incubator at 37 °C by further adding components in Table 3 below to a RPMI1640 (Invitrogen Gibco, Grand Island, NY) nutrient medium containing 10% fetus bovine serum (FBS; Hyclone, Logan, UT) after isolation of naive T cells from the spleen of a mouse.

**[Table 3]**

| Differentiated cell | Composition |
|---|---|
| Th0 | anti-CD3, anti-CD28 |
| Th1 | IL-12, anti-IL-4 antibody |
| Th2 | IL-4, anti-IFNβ |
| Th17 | IL-6, TGFβ, anti-IFNβ, anti-IL-4 |
| iTreg | IL-2, TGFβ |

### [Preparation Example 21 Treg isolation according to expression level of Lrig-1 protein

Naive T cells were differentiated into iTreg cells by isolating naive T cells from the spleen of a Foxp3-GFP KI mouse, and culturing the isolated naive T cells in the iTreg cell composition shown in Table 1 of Preparation Example 1 for 4 to 6 days. Afterward, the iTreg cells were stained with a Lrig-1 PE antibody (R&D Systems FAB3688P), and then iTreg Lrig-1^{low} and iTreg Lrig-1^{high} cultured for 4 days, and iTreg Lrig-1^{low} cultured for 6 days were isolated according to the expression level of Lrig-1 in FOXP3+ cells using FACS.

### [Example 11 Analysis of Lrig-1 structure

To produce an antibody specific for a Lrig-1 protein, which is a surface protein of Treg cells, the 3D structure of an extracellular domain of the Lrig-1 protein was predicted.

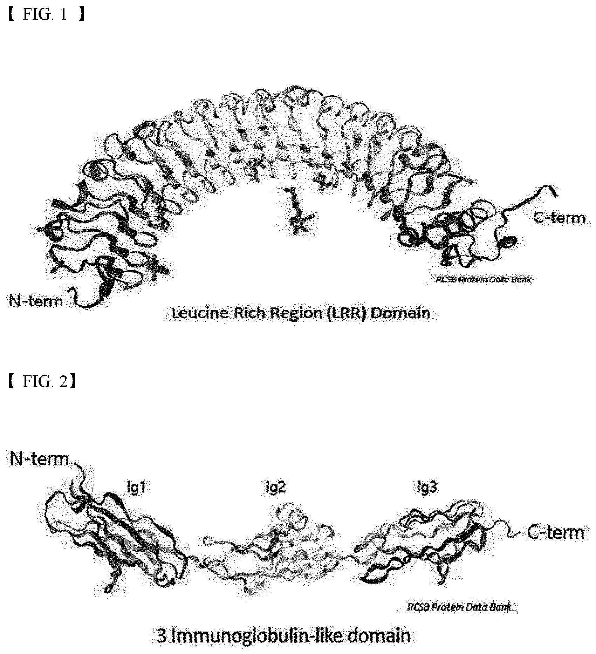

First, to predict the base sequence of an epitope, a 3D structure was predicted using Uniprot (http://www.uniprot.org) and an RCSB Protein Data Bank (http://www.rcsb.org/pdb) tool to confirm the structure of the extracellular domain (ECD) of the Lrig-1 protein, and the results are shown in FIGS. 1 and 2.

As shown in FIG. 1, there were a total of 15 leucine rich regions of LRR1 to LRR15 in the Lrig-LRR domain (the sequence of amino acids 41 to 494) among the ECDs of the Lrig-1 protein. Each of the LRR domains consisted of 23 to 27 amino acids, and included 3 to 5 leucines.

In addition, as shown in FIG. 2, there were three immunoglobulin-like domains at positions 494 to 781 in the amino acid sequence of the Lrig-1 protein of the ECD of the Lrig-1 protein.

### [Preparation Examples 1 to 81 Production of monoclonal antibody specific for Lrig-1 protein

An antibody, which is a binding molecule binding to a Lrig-1 protein according to the present invention, was produced.

To produce the antibody, Lrig-1 protein-expressing cells were produced. Specifically, a DNA fragment corresponding to SEQ ID NO: 2 and pcDNA (Hygro) were cleaved using a cleavage enzyme, and incubated at 37 °C for ligation, thereby producing pcDNA into which the DNA sequence of the Lrig-1 protein was inserted. The pcDNA into which the sequence of SEQ ID NO: 2 was inserted was transfected into L cells such that the Lrig-1 protein could be expressed on the surface of the L cells.

The amino acid sequences of a light chain and a heavy chain capable of binding to Lrig-1 expressed on the cell surface were selected from a human scFv library, resulting in obtaining a total of 8 heavy chains and light chains.

The amino acid sequences of the selected heavy chains and light chains were fused with a mlgG2a Fc region or human IgG1, thereby producing monoclonal antibodies corresponding to Preparation Examples 1 to 5. The sequences of the monoclonal antibodies are shown in Table 4 below.

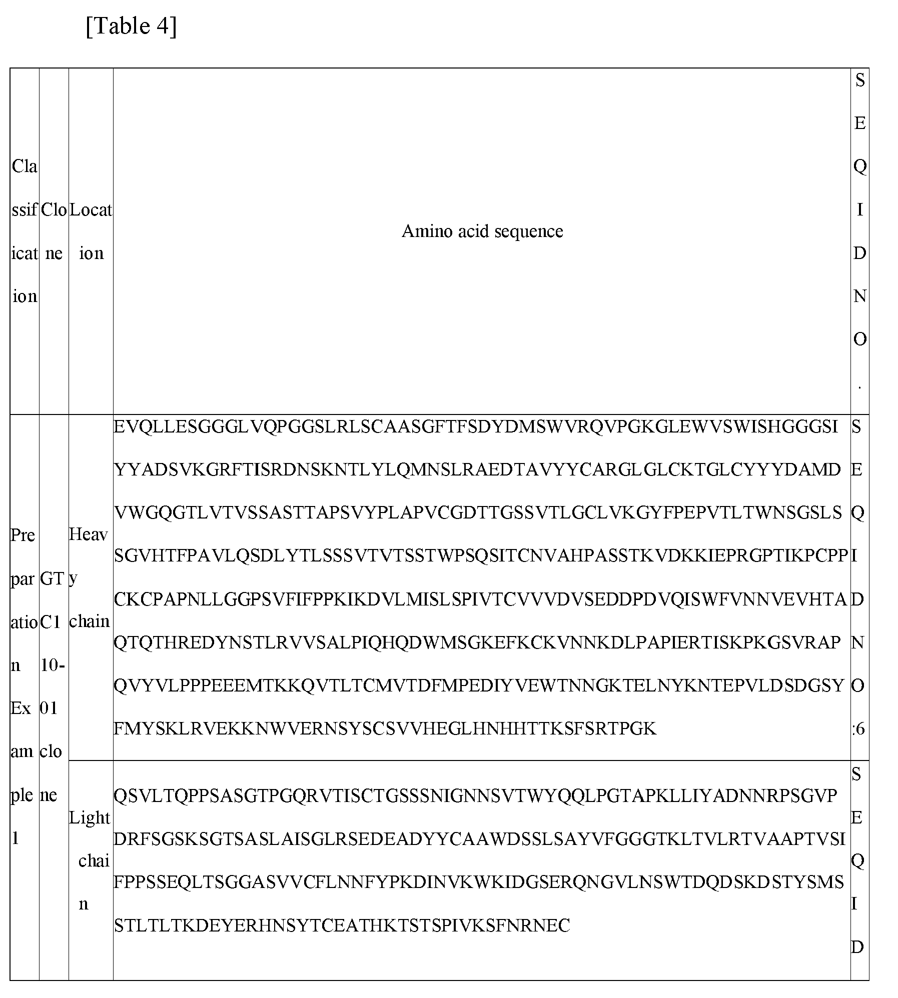

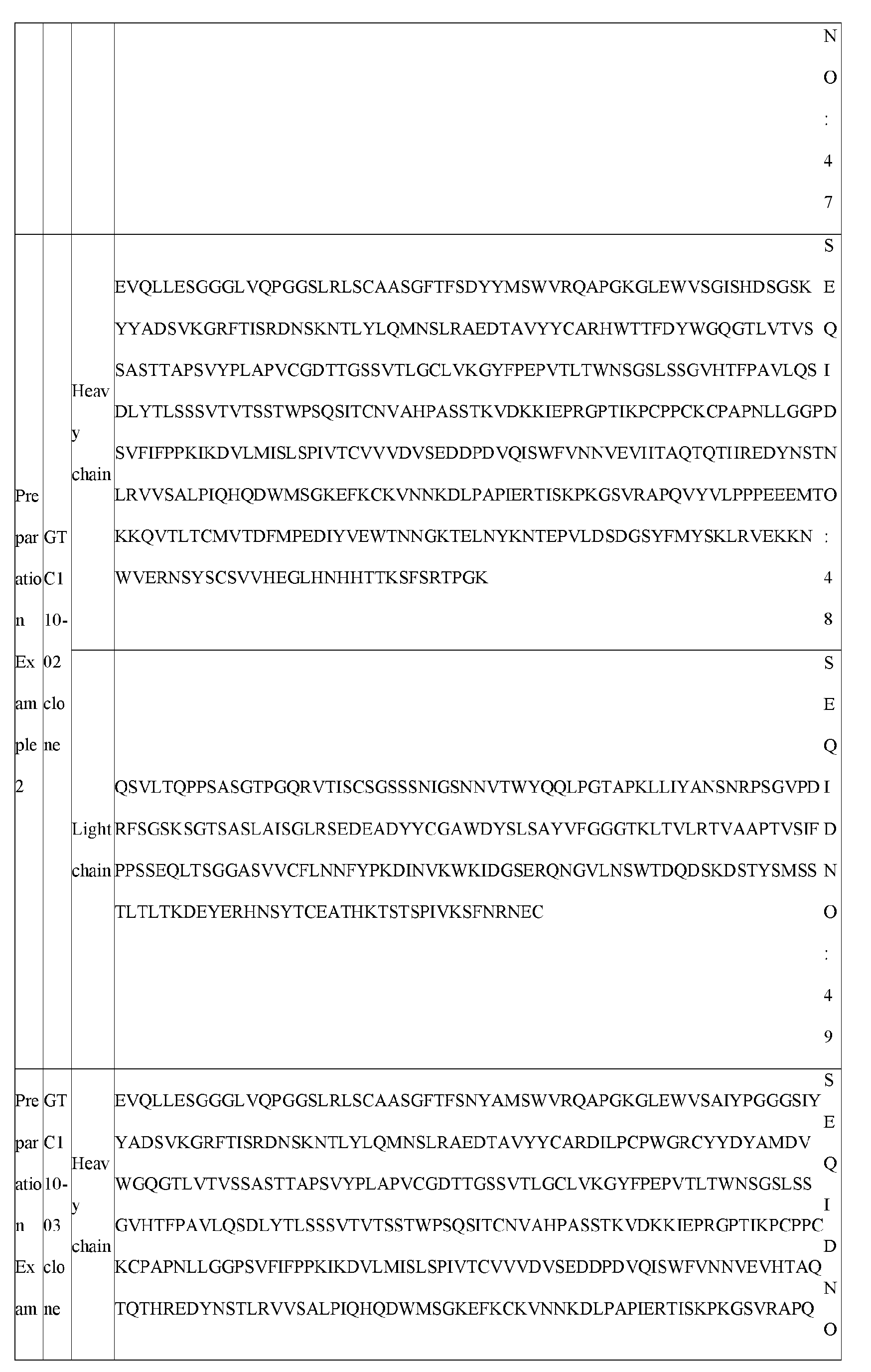

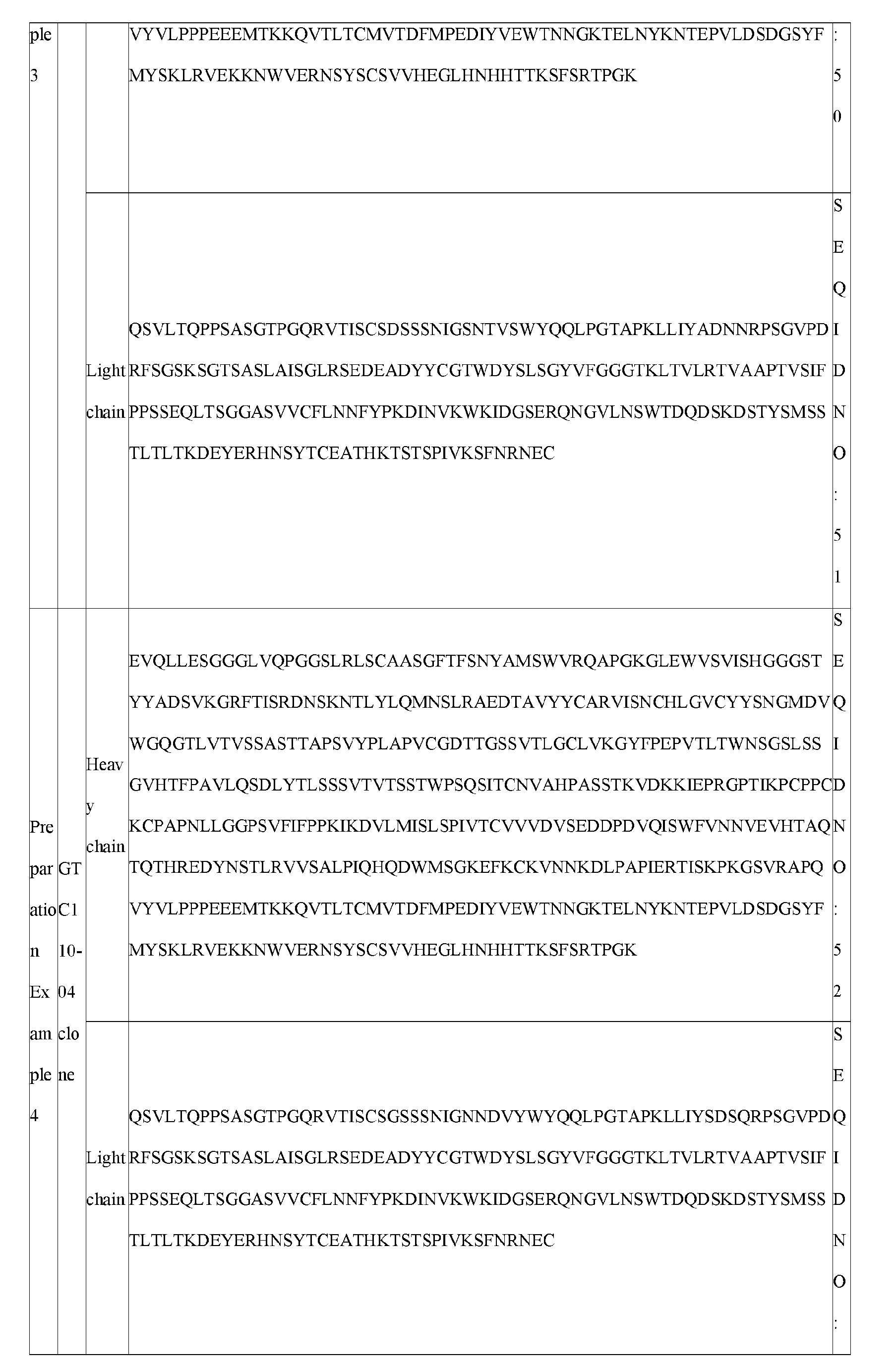

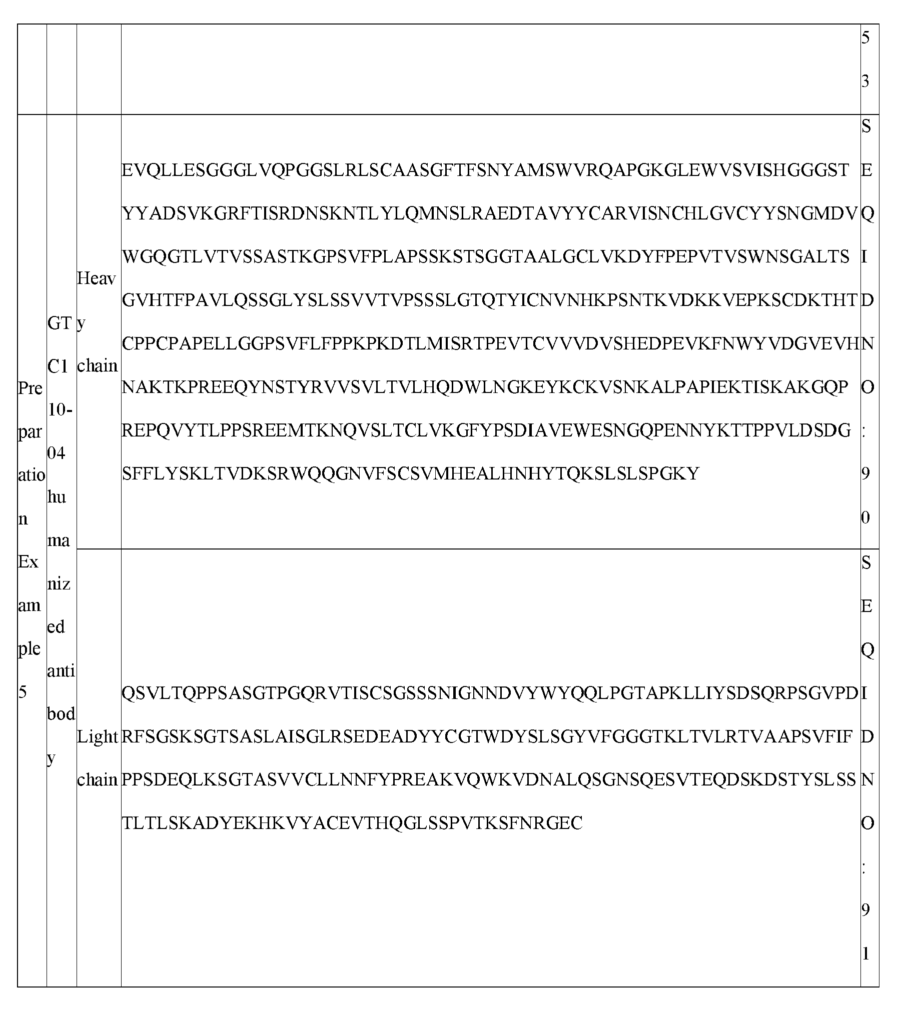

### [Example 21 Confirmation of specific expression of Lrig-1 mRNA in Treg cells

It was verified whether a Lrig-1 protein could serve as a Treg-specific biomarker.

For verification, CD4⁺ T cells were isolated from a mouse's spleen by magnet-activated cell sorting (MACS) using CD4 beads. Subsequently, Treg (CD4⁺CD25⁺ T) and non-regulatory T (CD4⁺CD25⁻ T) cells were isolated by FACS using CD25 antibodies. From each type of cells and the cells isolated in Preparation Example 1, mRNAs were extracted using Trizol, and gDNA was removed from the genomic RNA using a gDNA extraction kit (Qiagen) according to a manufacturer's protocol. A BDsprint cDNA synthesis kit (Clonetech) was used to synthesize cDNA from the gDNA-removed mRNA.

To quantify the expression level of Lrig-1 mRNA from the cDNA, real time polymerase chain reaction (real time PCR) was performed.

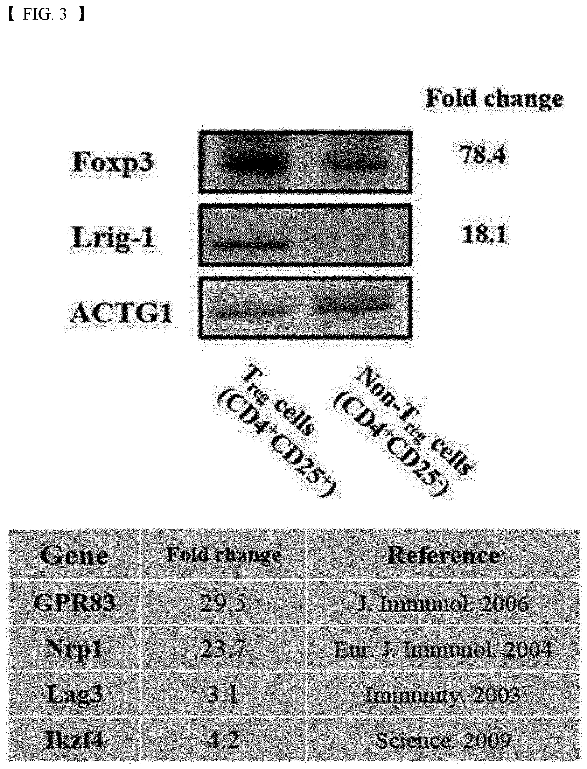

The real time PCR was performed with primers shown in Table 5 below using SYBR Green (Molecular Probes) according to a manufacturer's protocol for 40 cycles of 3 minutes at 95 °C, 15 seconds at 61 °C, and 30 seconds at 72 °C, and relative gene expression levels were calculated using a ΔΔCT method and then normalized by HPRT. The results are shown in FIGS. 3 to 6.

**[Table 5]**

| Primer | SEQ ID NO: | Sequence |
|---|---|---|
| Rat Lrig-1 | SEQ ID NO: 80 | Forward 5' - GAC GGA ATT CAG TGA GGA GAA CCT - 3' |
| | SEQ ID NO: 81 | Reverse 5' - CAA CTG GTA GTG GCA GCT TGT AGG - 3' |
| Rat Lrig-2 | SEQ ID NO: 82 | Forward 5' - TCA CAA GGA ACA TTG TCT GAA CCA- 3' |
| | SEQ ID NO: 83 | Reverse 5' - GCC TGA TCT AAC ACA TCC TCA- 3' |
| Rat Lrig-3 | SEQ ID NO: 84 | Forward 5' - CAG CAC CTT GAG CTG AAC AGA AAC - 3' |
| | SEQ ID NO: 85 | Reverse 5' - CCA GCC TTT GGT AAT CTC GGT TAG - 3' |
| Rat FOXP3 | SEQ ID NO: 86 | Forward 5' - CTT TCA CCT ATC CCA CCC TTA TCC - 3' |
| | SEQ ID NO: 87 | Reverse 5' - ATT CAT CTA CGG TCC ACA CTG CTC - 3' |
| ACTG1 | SEQ ID NO: 88 | Forward 5' - GGC GTC ATG GTG GGC ATG GG - 3' |
| | SEQ ID NO: 89 | Reverse 5' - ATG GCG TGG GGA AGG GCG TA - 3' |

As shown in FIG. 3, it can be seen that, compared to the non-regulatory T (CD4⁺CD25⁻ T) cells, in Treg (CD4⁺CD25⁺ T) cells, the Lrig-1 expression was 18.1-fold higher. This was an approximate 10-fold higher level compared to the conventionally known Treg markers Lag3 and Ikzf4.

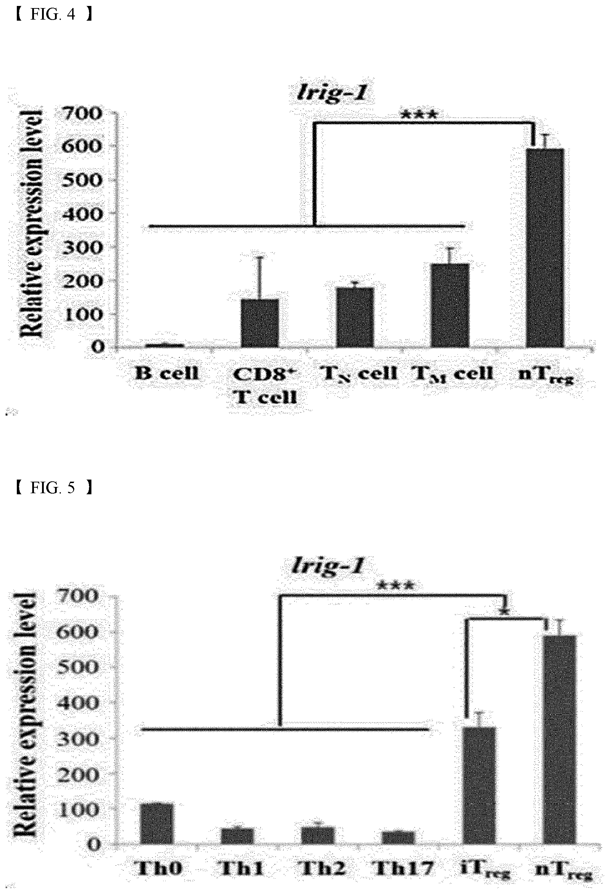

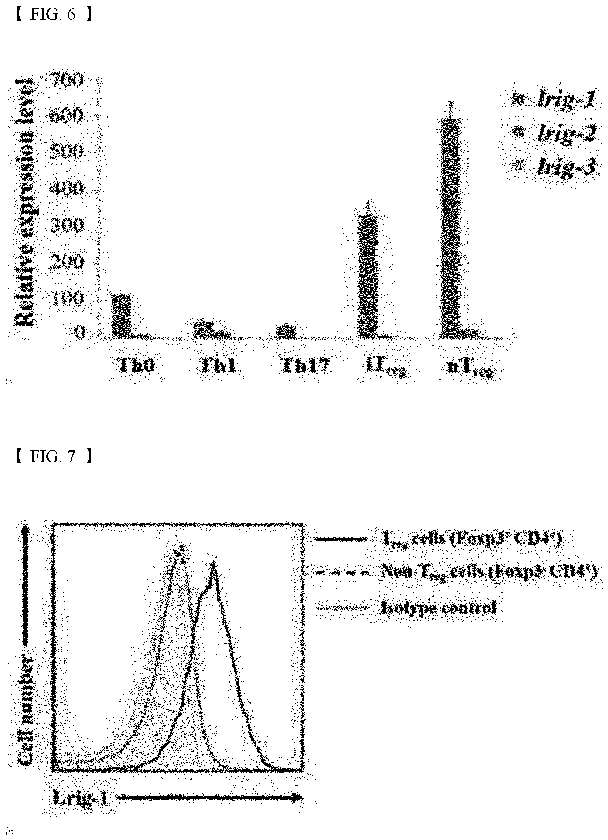

In addition, as shown in FIGS. 4 and 5, compared to a different type of immune cells, for example, Treg cells, particularly, differentiation-induced Treg cells (iTreg cells), in naturally isolated Treg cells ((nTreg cells), Lrig-1 mRNA expression was significantly higher. In addition, as shown in FIG. 6, among mRNAs of Lrig-1, Lrig-2 and Lrig-3 corresponding to the Lrig family, the mRNA expression level of Lrig-1 was the highest.

From the above result, it can be seen that the Lrig-1 protein according to the present invention is specifically expressed in Treg cells, particularly, naturally-occurring Treg cells.

### [Example 31 Confirmation of specific expression of Lrig-1 protein in Treg cells

It was confirmed whether the Lrig-1 protein expressed from the Lrig-1 mRNA is specifically expressed only in Treg cells.

CD4⁺ T cells were isolated from the spleen of a FOXP3-RFP-knock-in mouse prepared by binding of a red fluorescence protein (RFP) to a Treg-specific transcription factor FOXP3 promoter through MACS with CD4 beads. Subsequently, Treg (CD4⁺RFP⁺ T) cells and non-regulatory T (CD4⁺RFP⁻ T) cells were isolated through FACS using an RFP protein. Each type of cells were stained with a purchased Lrig-1 antibody, and a negative control was stained with an isotype, and then subjected to FACS to measure the expression levels of the Lrig-1 protein. The result is shown in FIG. 7.

As shown in FIG. 7, the non-regulatory T cells represented by a dotted line showed almost the same Lrig-1 protein expression level as the negative control, whereas there were many Treg cells with a higher Lrig-1 protein expression level.

From the above result, it can be seen that the Lrig-1 protein according to the present invention is specifically expressed in Treg cells.

### [Example 41 Confirmation of Lrig-1 protein-specific expression on Treg cell surface

In order to be a target for cell therapy, the Lrig-1 protein has to be expressed on the Treg cell surface for more effective target therapy, and thus it was confirmed whether the Lrig-1 protein is expressed on the Treg cell surface.

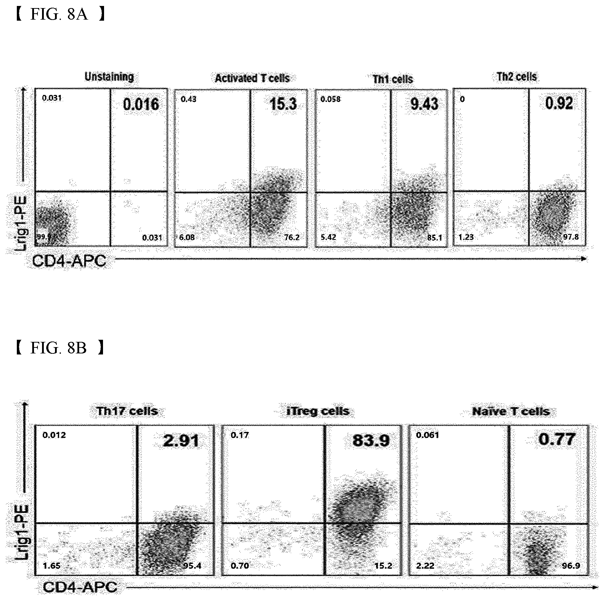

Each T cell subset differentiated in Preparation Example 1 was stained with anti-CD4-APC and anti-Lrig-1-PE antibodies, and Lrig-1 protein expression levels on the cell surface were measured by FACS. The results are shown in FIGS. 8A and 8B.

As shown in FIGS. 8A and 8B, in activated T cells, Th1 cells, Th2 cells, Th17 cells and naive T cells, the Lrig-1 protein was expressed at 0.77 to 15.3, whereas, in iTreg cells, the Lrig-1 protein was highly expressed at 83.9.

From the above result, it can be seen that the Lrig-1 protein according to the present invention is not only specifically expressed in Treg cells, but also more highly expressed on the surface of Treg cells.

### [Example 51 Evaluation of Lrig-1 protein binding ability of antibody according to the present invention

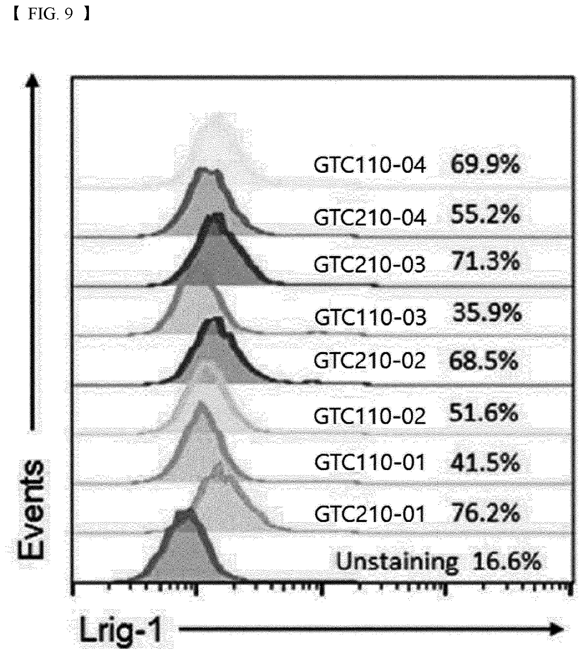

To confirm whether the monoclonal antibodies according to the present invention produced in Preparation Examples 1 to 4 recognize the Lrig-1 protein well, each type of antibodies produced in Preparation Examples 1 to 4 were bound to L cells in which the Lrig-1 protein is stably expressed, and then secondary antibodies which are able to recognize a mouse antibody and conjugated with eFlour 670 were added, followed by analysis of the binding ability of the monoclonal antibodies to the Lrig-1 protein by FACS. The result is shown in FIG. 9.

As shown in FIG. 9, it can be confirmed that all of the antibodies produced in Preparation Example 1 to 8 specific for the Lrig-1 protein according to the present invention effectively recognized the Lrig-1 protein present on the surface of the L cells and were bound thereto.

### [Example 61 Epitope determination of Lrig-1 protein according to the present invention

To find a binding epitope of the GTC110-04 clonal antibody of Preparation Example 4, which is present in LRR and Ig-like domains in the Lrig-1 protein present on the surface of Treg cells, the following experiment was performed.

### 1. Preparation of experiment

### (1) Microarray

To prevent peptide cleavage in the Lrig-1 protein, a GSGSGSG linker was elongated at each of the C- and N-termini of the Lrig-1 protein. The elongated antigen sequence was translated to 15 amino acids due to a peptide-peptide overlap of 14 amino acids. As a microarray result for the final *LRIG1* peptide, 1,091 different peptides were identified as duplicates, and framed by an additional HA (YPYDVPDYAG, 102 spots) control peptide.

### (2) Experimental materials

Sample: GTC110-04 monoclonal antibody of Preparation Example 4
Washing buffer: PBS with 0.05% Tween 20 (pH 7.4) (3 times, 10 seconds after each culture)
Blocking buffer: Rockland blocking buffer MB-070 (30 minutes before the first assay)
Culture buffer: washing buffer to which 10% blocking buffer was added
Assay conditions: an antibody concentration in the culture buffer is adjusted to 1 µg/ml, 10 µg/ml and 100 µg/ml; cultured for 16 hours at 4 °C, and stirred at 140 rpm
Secondary antibody: goat anti-human IgG (H+L) DyLight680 (0.2 µg/ml); stained for 45 minutes in the culture buffer at room temperature
Control antibody: mouse monoclonal anti-HA (12CA5) DyLight800 (0.5 µg/ml); stained for 45 minutes in the culture buffer at room temperature
Scanner: LI-COR Odyssey Imaging System; scanning offset: 0.65 mm, resolution: 21 µm, scanning intensity: 7/7 (red = 700 nm/green = 800 nm)

### 2. Experimental method

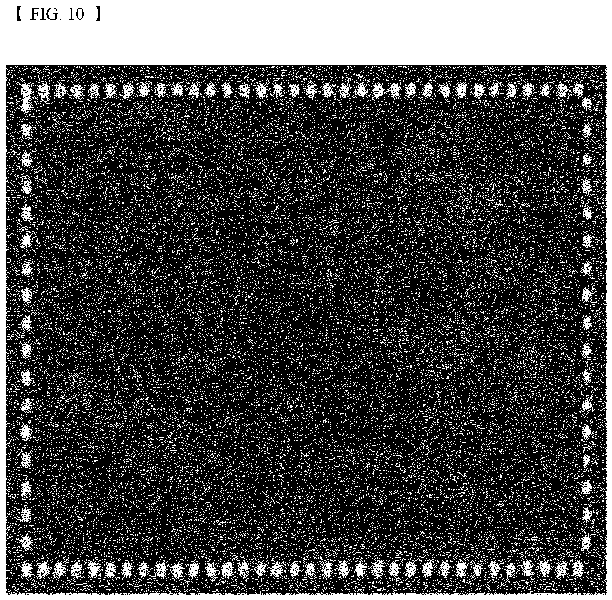

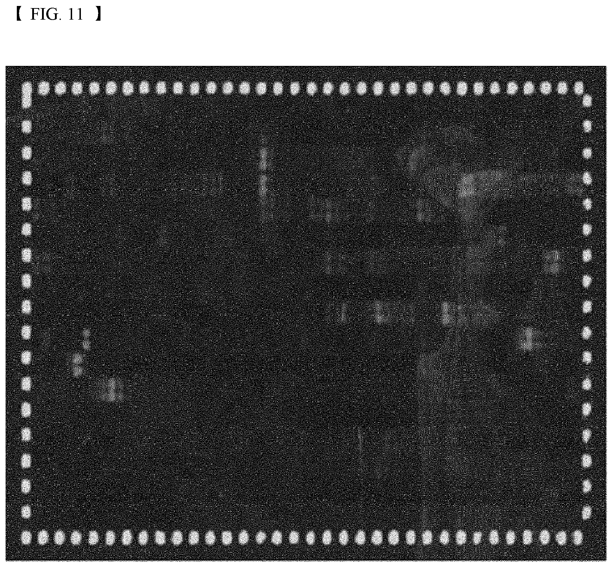

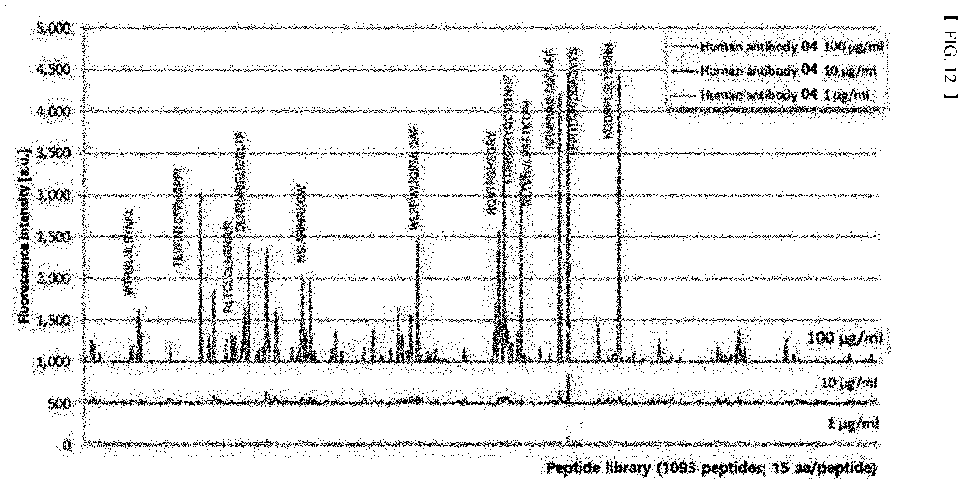

To confirm whether an interaction can be made with an antigen-derived peptide capable of causing interference in the main assay, pre-staining of microarray replicates of a Lrig-1 peptide was performed using the secondary antibody and the control antibody in the culture buffer. Subsequently, additional culture of the microarray replicates of another Lrig-1 peptide was performed in the culture buffer, along with the GTC110-04 monoclonal antibodies (1 µg/ml, 10 µg/ml and 100 µg/ml) of Preparation Example 4, followed by staining with the secondary antibody and the control antibody. Read-out was performed at a scanning intensity of 7/7 (red/green) using an LI-COR Odyssey imaging system. The quantification of spot intensity and peptide annotation were presented as 16-bit gray scale TIFF files. Microarray images were analyzed using a PepSlide^{®} Analyzer. The result is shown in FIGS. 10 to 12, and the analyzed epitope sequences are shown in Table 6 below.

**[Table 6]**

| Sequence list | Sequence data |
|---|---|
| SEQ ID NO: 68 | WTRSLNLSYNKL |
| SEQ ID NO: 69 | TEVRNTCFPHGPPI |
| SEQ ID NO: 70 | RLTQLDLNRNRIR |
| SEQ ID NO: 71 | DLNRNRIRLIEGLTF |
| SEQ ID NO: 72 | NSIARIHRKGW |
| SEQ ID NO: 73 | WLPPWLIGRMLQAF |
| SEQ ID NO: 74 | RQVTFGHEGRY |
| SEQ ID NO: 75 | FGHEGRYQCVITNHF |
| SEQ ID NO: 76 | RLTVNVLPSFTKTPH |
| SEQ ID NO: 77 | RRMHVMPDDDVFF |
| SEQ ID NO: 78 | FFITDVKIDDAGVYS |
| SEQ ID NO: 79 | KGDRPLSL TERHH |

### 3. Experimental result

As shown in FIGS. 10 to 12, it can be confirmed that a monoclonal antibody (GTC110-04) which effectively treats cancer by specifically binding to a Lrig-1 protein present on Treg cells exhibits the above-described functions by specifically binding to epitopes, which consist of SEQ ID NOs: 68 to 79, in the Lrig-1 protein. Moreover, the epitopes represented by SEQ ID NOs: 66 and 68 may have a leucine rich repeat in common, and specifically, a consensus sequence that can be represented by an amino acid sequence of Formula 1 below may be included at the 5^{th} amino acid position from the N-terminus.

[Formula 1] Lx¹Lx²x³N

In Formula 1,
x¹ to x³ may each be independently a neutral amino acid, an acidic amino acid, a basic amino acid or an aromatic amino acid. Here, the neutral amino acid may be glycine (G), alanine (A), valine (V), leucine (L), isoleucine (I), serine (S) or threonine (T), the acidic amino acid may be aspartic acid (D), glutamic acid (E), asparagine (N) or glutamine (Q), the basic amino acid may be lysine (K), arginine (R) or histidine (H), and the aromatic amino acid may be phenylalanine (F) or tyrosine (Y).

### [Example 7] Regulation of signaling pathway in Treg cells by antibody according to the present invention

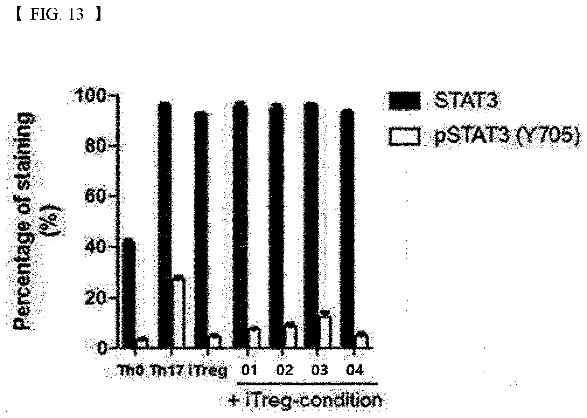

To analyze how the monoclonal antibodies according to the present invention, produced in Preparation Examples 1 to 4, affect the signaling pathway in Treg cells through the Lrig-1 protein, the Lrig-1 protein present on the Treg cell surface was stimulated by treating the Treg cells with each of the antibodies of Preparation Examples 1 to 4, a degree of tyrosine phosphorylation of a Stat3 protein present in the stimulated Treg cells was analyzed by phosphotyrosine immunoblotting. The result is shown in FIG. 13.

As shown in FIG. 13, it can be confirmed that monoclonal antibodies (GTC110-01, GTC110-02, GTC110-03 and GTC110-04), which are binding molecules specifically binding to the Lrig-1 protein according to the present invention, continuously maintained and reduced Stat3 phosphorylation at the same level as iTreg cells.

### [Example 8] Confirmation of Treg cell function according to Lrig-1 expression level

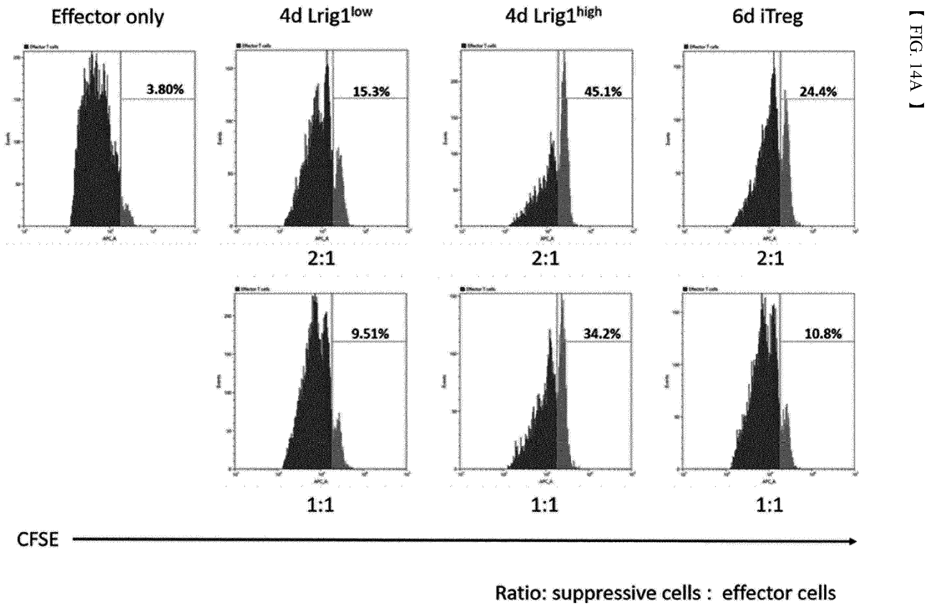

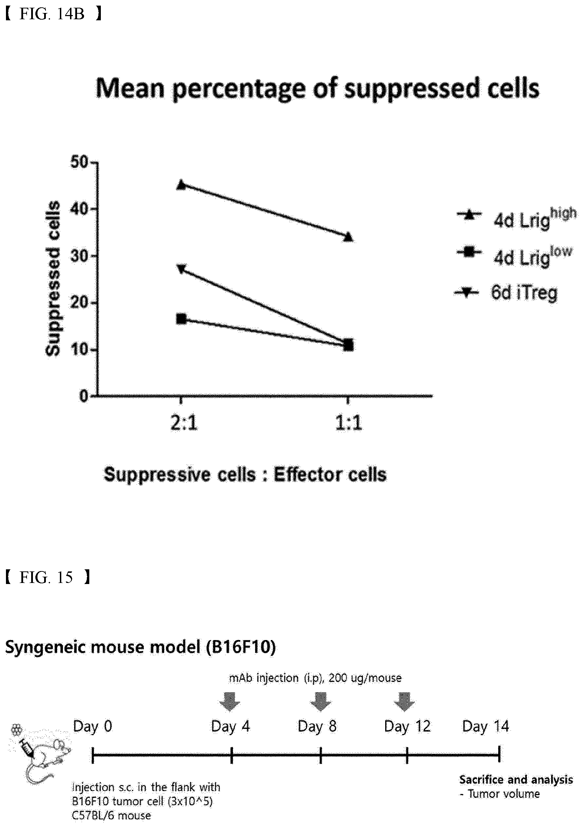

iTreg Lrig-1^{low} cultured for 4 days (hereinafter, referred to as "4d iTreg Lrig-1^{low}"), iTreg Lrig-1^{high} cultured for 4 days (hereinafter, referred to as "4d iTreg Lrig-1^{high}") or iTreg Lrig-1^{low} cultured for 6 days (hereinafter, referred to as "6d iTreg Lrig-1^{low}"), which was isolated in Preparation Example 2, and effector T cells isolated from a C57BL/6 mouse were mixed at 1:1 or 2:1 and cultured, followed by analysis of a degree of suppressing the effector T cells by each type of Treg cells through FACS. The result is shown in FIGS. 14A and 14B.

As shown in FIGS. 14A and 14B, when the effector T cells were mixed with 4d iTreg Lrig-1^{low} and 6d iTreg Lrig-1^{low}, the functions of 15.3% (2:1) and 24.4% (2:1) of the effector T cells were suppressed, but when mixed with 4d iTreg Lrig-1^{high}, the functions of 45.1% (2:1) and 34.2% (1:1) of the effector T cells were suppressed.

Accordingly, Treg cells on the surface of which the Lrig-1 protein according to the present invention is expressed are activated Treg cells capable of suppressing effector T cells, and identified as tumor infiltrating lymphocytes (TILs). Therefore, the binding molecule specifically binding to the Lrig-1 protein expressed on the Treg cell surface may ultimately induce the death of cancer cells by the effector T cells by effectively suppressing the function of suppressing the effector T cells.

Moreover, it can be seen that this is a separate mechanism from the immune checkpoint evasion of tumor cells occurring between cancer cells and effector T cells, and very effectively applied to resistant cancer due to an immune checkpoint inhibitor by maintaining the activity of effector T cells, resulting in the prevention, improvement or treatment of cancer.

### [Example 9] Confirmation of therapeutic effect on immune checkpoint inhibitor-resistant cancer (1)

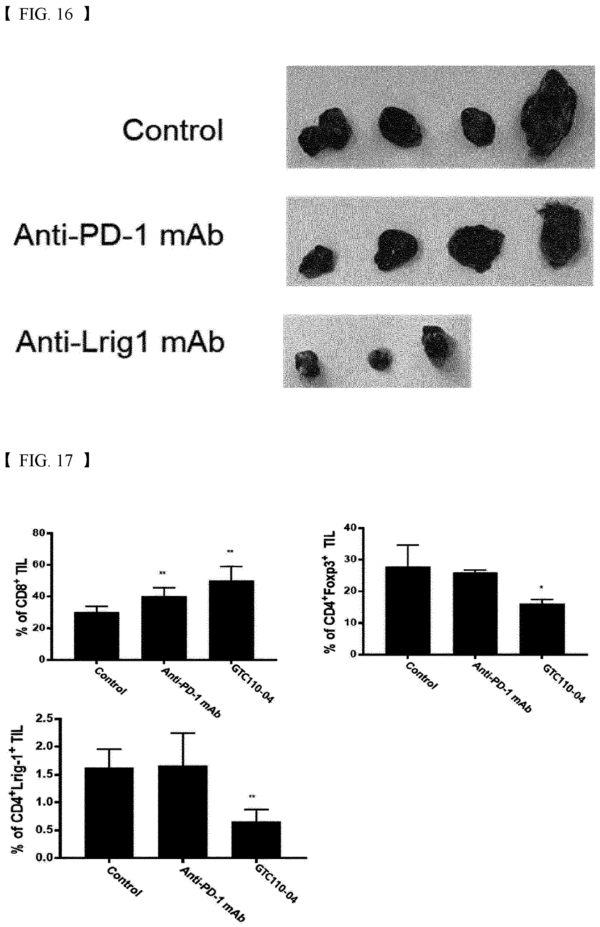

To compare the therapeutic effects on solid tumors between the monoclonal antibody (GTC110-04) according to the present invention, produced in Preparation Example, and a conventional immune checkpoint inhibitor, which is a PD-1 antibody, as shown in FIG. 15, 3 × 10⁵ B16F10 melanoma cells were administered to the back of a mouse through subcutaneous injection, and on day 4, 8 and 12, 200 µg of the antibody (GTC110-04) of Preparation Example 8 or the PD-1 antibody was intraperitoneally injected. After the melanoma cell transplantation, a change in volume of the tumor over time was measured. The result is shown in FIG. 16.

As shown in FIG. 16, when the PD-1 antibody was injected, the tumor size was only similar to that of the negative control, whereas when the monoclonal antibody (GTC110-04), which is a binding molecule specifically binding to the Lrig-1 protein according to the present invention, was injected, the tumor size was significantly reduced compared to the negative control.

Therefore, it can be seen that the monoclonal antibody specific for the Lrig-1 protein according to the present invention may suppress the growth of solid tumor cells with immune checkpoint inhibitor resistance, which cannot be treated by PD-1, and thus solid tumors with immune checkpoint inhibitor resistance may be effectively prevented, improved or treated.

### [Example 10] Confirmation of therapeutic effect on immune checkpoint inhibitor-resistant cancer (2)

The cell distribution of the TILs in a melanoma animal model in Example 9 was investigated.

Specifically, tumor tissue was removed from the melanoma mouse model, and then put into a 6-well plate containing 2 mL PBS (containing 2% FBS), followed by fine grinding thereof using an e-tube. Here, when the size of the tumor tissue was too large, the tissue was first cut with scissors. The finely ground tumor tissue and all tissues attached to the wall of the e-tube filtered through a strainer were put into a 50 mL tube, adjusted to have a final volume of 20 mL, and centrifuged at 4 °C and 2000 rpm for 10 minutes. Afterward, the resulting supernatant was removed through suction, 3 mL of RBC lysis buffer was added, and reacted at room temperature for 30 seconds, followed by inactivation by adding 2% RPMI. Subsequently, the supernatant was removed by centrifugation, and a resuspended pellet was filtered through a strainer, followed by centrifugation. Afterward, a pellet resuspended using 8 mL of red 44% Percoll (Percoll : RPMI = 11 : 14) was transferred to a 15 mL tube, 5 mL of pure 67% Percoll (Percoll : PBS = 67 : 33) was slowly added to the lower layer of the 15 mL tube using a Pasteur pipette. The 15 mL tube was subjected to centrifugation under a no brake condition at 4 °C and 2000 rpm for 20 minutes, up to about 1 mL of the buffy coat was removed, and then the buffy coat was isolated and transferred to a 15 mL tube. Afterward, resuspension was performed in the 15 mL tube containing the buffer coat using 2% FBS-containing PBS, and then the number of TILs was measured.

The TILs obtained as described above were seeded in each well of a roundbottom 96-well cell culture plate at 1 × 10⁶ cells/200 µL, and centrifuged at 2500 rpm for 2 minutes. Afterward, the resulting supernatant was removed, antibodies targeting primary markers (CD4, CD8 and Lrig-1) diluted in PBS were dispensed in each well at 50 µL and then well mixed by pipetting. Subsequently, light was blocked on the plate using foil and the plate was incubated in a refrigerator at 4 °C for 30 minutes, and 150 µL of PBS was further added to perform centrifugation. Afterward, 80 µL of a fixation/perm reagent was dispensed into each well, incubated at 4 °C for 30 minutes, and 120 µL of 1× perm buffer was added to perform centrifugation. Finally, 1 µL of a secondary marker (Foxp3) contained in 1× perm buffer was dispensed into each well at 50 µL and incubated at 4 °C for 30 minutes, and cells obtained by centrifugation were resuspended in 200 µL of PBS, followed by measurement by FACS. The result is shown in FIG. 17.

As shown in FIG. 17, when the PD-1 antibody was injected, the number of CD8+ T cells infiltrating a tumor was insignificantly increased compared to that of a control, whereas when the monoclonal antibody (GTC110-04), which is the binding molecule specifically binding to the Lrig-1 protein according to the present invention, was injected, compared to a negative control, the number of CD8+ T cells infiltrating a tumor more than doubled. Further, it was confirmed that the number of CD8+ T cells infiltrating a tumor was not changed by the PD-1 antibody, whereas the number of CD8+ T cells infiltrating a tumor was significantly decreased by the monoclonal antibody (GTC110-04).

From the above result, it can be seen that as the binding molecule specifically binding to the Lrig-1 protein according to the present invention significantly reduced the number of CD4+Foxp3+ T cells infiltrating a tumor, the function of effector T cells was improved, thereby more effectively exhibiting a synergistic effect on prevention or treatment of immune checkpoint inhibitor-resistant cancer.

While the present invention has been described in detail with reference to exemplary embodiments of the present invention, it will be obvious to those of ordinary skill in the art that various modifications and variations are possible without departing from the technical spirit of the present invention described in the claims.

### [Industrial Applicability]

A binding molecule, and preferably, an antibody, which is specific for a Lrig-1 protein, according to the present invention may effectively prevent, improve or treat immune evasion cancer, that is, cancer with resistance to an immune checkpoint inhibitor by very effectively blocking a phenomenon in which the function of effector T cells is inhibited by regulatory T cells (Treg cells) by inhibiting the function of Treg cells in which the Lrig-1 protein is expressed at a high level, that is, Treg cells belonging to TILs or activated Treg cells.

## Claims

1. A pharmaceutical composition for preventing or treating immune checkpoint inhibitor-resistant cancer, comprising a binding molecule specifically binding to a leucine-rich and immunoglobulin-like domains 1 (Lrig-1) protein as an active ingredient.

2. The pharmaceutical composition of claim 1, wherein the Lrig-1 protein consists of an amino acid sequence represented by SEQ ID NO: 1 or 3.

3. The pharmaceutical composition of claim 1, wherein the Lrig-1 protein is encoded by a polynucleotide represented by SEQ ID NO: 2 or 4.

4. The pharmaceutical composition of claim 1, wherein the Lrig-1 protein is present on the surface of regulatory T (Treg) cells.

5. The pharmaceutical composition of claim 4, wherein the Treg cells are tumor infiltrating lymphocytes (TIL) or activated Treg cells.

6. The pharmaceutical composition of claim 1, wherein the binding molecule is an antibody or a fragment thereof.

7. The pharmaceutical composition of claim 6, wherein the antibody is a chimeric antibody, a humanized antibody, a human antibody, a bivalent antibody, a bispecific antibody, a minibody, a domain antibody, a bispecific antibody, an antibody mimetic, a unibody, a diabody, a triabody, a tetrabody or a fragment thereof.

8. The pharmaceutical composition of claim 1, further comprising:
an anticancer agent.

9. The pharmaceutical composition of claim 1, wherein the cancer is a solid tumor.

10. The pharmaceutical composition of claim 1, wherein the cancer is stomach cancer, liver cancer, glioblastoma, ovarian cancer, colorectal cancer, head and neck cancer, bladder cancer, renal cell carcinoma, breast cancer, metastatic cancer, prostate cancer, pancreatic cancer, melanoma or lung cancer.

11. A pharmaceutical composition for preventing or treating immune checkpoint inhibitor-resistant cancer, comprising:
an antibody-drug conjugate comprising an antibody or antigen-binding fragment specifically binding to a leucine-rich and immunoglobulin-like domains 1 (Lrig-1) protein and a drug, as an active ingredient.

12. The pharmaceutical composition of claim 11, wherein the cancer is a solid tumor.

13. The pharmaceutical composition of claim 11, wherein the cancer is stomach cancer, liver cancer, glioblastoma, ovarian cancer, colorectal cancer, head and neck cancer, bladder cancer, renal cell carcinoma, breast cancer, metastatic cancer, prostate cancer, pancreatic cancer, melanoma or lung cancer.

14. A method of preventing or treating immune checkpoint inhibitor-resistant cancer, comprising:
administering a binding molecule specifically binding to a Lrig-1 protein into a subject at a pharmaceutically effective amount.

15. A method of preventing or treating immune checkpoint inhibitor-resistant cancer, comprising:
administering an antibody-drug conjugate comprising an antibody or antigen-binding fragment specifically binding to a leucine-rich and immunoglobulin-like domains 1 (Lrig-1) protein and a drug into a subject at a pharmaceutically effective amount.
